# EUROPÄISCHE PATENTSCHRIFT

(11) **EP 0 532 525 B1**
(45) Veröffentlichungstag und Bekanntmachung des Hinweises auf die Patenterteilung: **16.08.1995**
(21) Anmeldenummer: 91909227.0
(22) Anmeldetag: 10.05.1991
(51) Int. Cl.: A61K 47/48, C12N 15/87

(54) **NEUE PROTEIN-POLYKATION-KONJUGATE**
NEW PROTEIN-POLYCATION CONJUGATES
NOUVEAUX CONJUGUES DE PROTEINES ET DE POLYCATIONS

(30) Priorität: 18.05.1990 AT 1110/90; 29.03.1991 DE 4110410
(43) Veröffentlichungstag der Anmeldung: 24.03.1993
(73) Patentinhaber: BOEHRINGER INGELHEIM INTERNATIONAL GmbH, 55218 Ingelheim am Rhein (DE); GENENTECH, INC., South San Francisco California 94080 (US)
(72) Erfinder: BIRNSTIEL, Max, L., A-1100 Wien (AT); COTTEN, Matthew, A-1130 Wien (AT); WAGNER, Ernst, A-2103 Langenzersdorf (AT)
(74) Vertreter: Laudien, Dieter, Dr.
(86) Internationale Anmeldenummer: EP9100875
(87) Internationale Veröffentlichungsnummer: WO9117773

(56) Entgegenhaltungen:
- EP-A- 0 388 758
- WO-A-88/05077
- WO-A-90/01951
- Cellular & Molecular Biology, Band 28, Nr. 1, 1982, Pergamon Press LTD., (OXford, GB), J.C. Stavridis et al.: "Use of transferrin as a gene-carrier to the erythroid cells of the marrow", Seiten 15-18, siehe Seite 15,
- Proc. Natl. Acad. Sci. Band 87, 4. Mai 1990, (Washington D.C., US), E. Wagner et al.: "Transferrin-polycation conjugates as carriers for DNA uptake into cells", Seiten 3410-3414, siehe SEite 3410, Zusammenfassung
- THE JOURNAL OF BIOLOGICAL CHEMISTRY, Band 263, Nr. 29, 15. Oktober 1988, The American Society for Biochemistry and Molecular Biology. Inc., (Baltimore, US), G.Y. Wu et al.:" Receptor-mediated gene delivery and expression in vivo", Seiten 14621-14624, siehe SEite 14621, Zusammenfassung (in der Anmeldung erwähnt)

## Beschreibung

Die Erfindung betrifft neue Protein-Polykation-Konjugate für den Transport von Nukleinsäuren in menschliche oder tierische Zellen.

Nukleinsäuren als therapeutisch wirksame Substanzen haben in den letzten Jahren zunehmend an Bedeutung gewonnen.

Antisense RNAs und -DNAs haben sich als wirksame Mittel für die selektive Inhibierung bestimmter Gensequenzen erwiesen. Ihre Wirkungsweise ermöglicht ihre Anwendung als Therapeutika zur Blockierung der Expression bestimmter Gene (wie deregulierter Oncogene oder viraler Gene) in vivo. Es wurde bereits gezeigt, daß kurze Antisense- Oligonukleotide in Zellen importiert werden und dort ihre inhibierende Wirkung ausüben können (Zamecnik et al., 1986), wenngleich ihre intrazelluläre Konzentration, u.a. wegen ihrer beschränkten Aufnahme durch die Zellmembran auf Grund der starken negativen Ladung der Nukleinsäuren, gering ist.

Ein weiterer Ansatz zur selektiven Inhibierung von Genen besteht in der Anwendung von Ribozymen. Auch hier besteht das Bedürfnis, eine möglichst hohe Konzentration von aktiven Ribozymen in der Zelle zu gewahrleisten, wofür der Transport in die Zelle einer der limitierenden Faktoren ist.

Es wurden bereits mehrere Lösungen vorgeschlagen, den Transport von Nukleinsäuren in lebende Zellen, der bei deren therapeutischer Anwendung einen der limitierenden Faktoren darstellt, zu verbessern.

Einer dieser Lösungswege besteht in direkten Modifikationen der Nukleinsäuren, z.B. durch Substitution der geladenen Phosphodiestergruppen durch ungeladene Gruppen. Eine weitere Möglichkeit der direkten Modifikation besteht in der Verwendung von Nukleosidanalogen.

Wenn auch einige dieser Vorschläge einen grundsätzlich vielversprechenden Ansatz der Lösung des Problems darstellen, weisen sie doch verschiedene Nachteile auf, z.B. geringere Bindung an das Zielmolekül, geringere Hemmwirkung, mögliche Toxizität.

Ein alternativer Ansatz zur direkten Modifikation der Oligonukleotide besteht darin, das Oligonukleotid als solches unverändert zu belassen und mit einer Gruppe zu versehen, die ihm die angestrebten Eigenschaften verleiht, z.B. mit Molekülen, die den Transport in die Zelle erleichtern.

Neben der Inhibierung von Genen besteht Bedarf an einem effizienten System für das Einführen von Nukleinsäure in lebende Zellen im Rahmen der Gentherapie. Dabei werden Gene in Zellen eingeschleust, um in vivo die Synthese therapeutisch wirksamer Genprodukte zu erzielen, z.B. um im Falle eines genetischen Defekts das fehlende Gen zu ersetzen. Beispiele für Anwendungsmöglichkeiten bei genetisch bedingten Erkrankungen, für deren Behandlung die Gentherapie einen erfolgversprechenden Ansatz darstellt, sind Hämophilie, beta-Thalassämie und "Severe Combined Immune Deficiency"(SCID), ein Syndrom, das durch einen genetisch bedingten Mangel des Enzyms Adenosindeaminase hervorgerufen wird. Die "klassische" Gentherapie beruht auf dem Prinzip, durch eine einmalige Behandlung eine dauernde Heilung zu erzielen. Daneben besteht jedoch Bedarf an Behandlungsmethoden, bei denen die therapeutisch wirksame DNA (oder auch mRNA) wie ein Medikament ("Gentherapeutikum") je nach Bedarf einmalig oder wiederholt verabreicht wird. Anwendungsmöglichkeiten für dieses Prinzip bestehen bei der Immunregulation, wobei durch Verabreichung funktioneller Nukleinsäure, die für ein sekretiertes Protein-Antigen oder für ein nicht-sekretiertes Protein-Antigen codiert, mittels einer Impfung eine humorale oder intrazelluläre Immunität erzielt wird. Beispiele für genetische Defekte, bei denen eine Verabreichung von Nukleinsäure, die für das defekte Gen codiert, in individuell auf den Bedarf abgestimmter Form verabreicht werden kann, sind Muskeldystrophie (Dystrophin-Gen), Cystische Fibrose (Transmembran-Regulations-Gen), Hypercholesterinämie (HDL-Rezeptor-Gen). Gentherapeutische Behandlungsmethoden sind weiters potentiell dann von Bedeutung, wenn Hormone, Wachstumsfaktoren oder cytotoxisch oder immunmodulierend wirkende Proteine im Organismus synthetisiert werden sollen.

Die bisher am weitesten fortgeschrittenen Technologien für die Anwendung von Nukleinsäuren im Rahmen der Gentherapie benutzen retrovirale Systeme für den Transfer von Genen in die Zelle (Wilson et al., 1990, Kasid et al., 1990). Die Verwendung von Retroviren ist jedoch problematisch, weil sie, zumindest zu einem geringen Prozentsatz, die Gefahr von Nebenwirkungen wie Infektion mit dem Virus (durch Rekombination mit endogenen Viren und mögliche anschließende Mutation zur pathogenen Form) oder Entstehung von Krebs in sich birgt. Außerdem ist die stabile Transformation der somatischen Zellen des Patienten, wie sie mit Hilfe von Retroviren erzielt wird, nicht in jedem Fall wünschenswert, weil dadurch die Behandlung, z.B. bei Auftreten von Nebeneffekten, nur mehr schwierig rückgängig gemacht werden kann.

Es wurde daher nach alternativen Methoden gesucht, um die Expression von nicht-replizierender DNA in der Zelle zu ermöglichen.

Für die Gentransformation von Säugetierzellen in vitro sind verschiedene Techniken bekannt, deren Anwendbarkeit in vivo jedoch beschränkt ist (dazu zählen das Einbringen von DNA mittels Liposomen, Elektroporation, Mikroinjektion, Zellfusion, DEAE-Dextran oder die Calciumphosphat-Präzipitationsmethode).

Neuere Bestrebungen, Methoden für den in vivo Gentransfer zu entwickeln, konzentrieren sich auf die Verwendung des kationischen Lipidreagens Lipofektin, von dem gezeigt werden konnte, daß ein mit Hilfe dieses Reagens injiziertes Plasmid im Organismus exprimiert wird (Nabel et al., 1990).

Eine weitere kürzlich entwickelte Methode benutzt Mikropartikel aus Wolfram oder Gold, an denen DNA absorbiert ist, mit denen die Zelle mit hoher Energie beschossen werden (Johnston, 1990, Yang et al., 1990). Expression der DNA konnte in verschiedenen Geweben nachgewiesen werden.

Ein in vivo anwendbares lösliches System, das DNA zielgerichtet in die Zellen befördert, wurde für Hepatozyten entwickelt und beruht auf dem Prinzip, Polylysin an ein Glykoprotein, auf das ein an der Hepatozytenoberfläche vorhandener Rezeptor anspricht, zu koppeln und daraufhin durch Zugabe von DNA einen löslichen Glykoprotein/Polylysin/DNA-Komplex zu bilden, der in die Zelle aufgenommen wird und nach Aufnahme des Komplexes in die Zelle die Expression der DNA-Sequenz ermöglicht (G.Y. Wu, C.H. Wu, 1987).

Dieses System ist spezifisch für Hepatozyten und wird hinsichtlich seiner Funktion durch den relativ gut charakterisierten Aufnahmemechanismus durch den Asialoglykoproteinrezeptor definiert.

Ein breiter anwendbares, effizientes Transportsystem benutzt den Transferrinrezeptor für die Aufnahme von Nukleinsäuren in die Zelle mittels Transferrin-Polykation-Konjugaten. Dieses System ist Gegenstand der Europäischen Patentanmeldung A1 388 758. Es konnte gezeigt werden, daß Transferrin-Polykation/DNA-Komplexe effizient in lebende Zellen aufgenommen und internalisiert werden, wobei als Polykationanteil der Komplexe Polylysin verschiedenen Polymerisationsgrades und Protamin verwendet wurden. Mit Hilfe dieses Systems wurde u.a. ein das erbB-Oncogen inhibierendes Ribozymgen in erbB-transformierte Hühnerzellen eingebracht und die erbB inhibierende Wirkung nachgewiesen.

Der vorliegenden Erfindung lag die Aufgabe zugrunde, ein System bereitzustellen, mit Hilfe dessen ein selektiver Transport von Nukleinsäuren in höhere eukaryotische Zellen, insbesondere Zellen der T-Zell-Abstammungslinie ("T-cell lineage") ermöglicht wird. (Im folgenden werden Zellen der T-Zell-Abstammungslinie der Einfachheit halber als T-Zellen bezeichnet. Darunter sind Vorläufer-T-Zellen sowie die daraus diversifizierenden Linien einschließlich der reifen T-Zellen zu verstehen).

T-Lymphozyten (T-Zellen) differenzieren im Thymus. Eine ihrer Aufgaben ist die Unterstützung der B-Zellen bei der Antigenantwort. Eines der Charakteristika von T-Zellen ist, daß sie kein freies Antigen erkennen, sondern Fragmente von Antigenen. T-Zellen erkennen ein solches Peptid-Antigen-Fragment auf der Oberfläche von Zielzellen durch einen T-Zell-Antigenrezeptor (TCR), der mit einem an ein MHC (major histocompatibility complex)-Molekül gebundenen Antigen in Wechselwirkung tritt. Die spezifische Antigenerkennung erfordert die Mitwirkung eines weiteren Rezeptors, CD4 oder CD8, mit nicht-polymorphen Regionen von MHC. Dieses Zusammenwirken von TCR und entweder CD4 oder CD8 mit einem MHC-Molekül auf Zielzellen ist für die Ausbildung der spezifischen Fähigkeiten von T-Zellen während der thymischen Entwicklung erforderlich und erlaubt die antigenspezifische Aktivierung reifer T-Zellen. T-Zellen, die mit Klasse I MHC Molekülen assoziiertes Antigen erkennen (vorwiegend Killerzellen), exprimieren CD8; Zellen, die Klasse II-assoziierte Antigene erkennen (vorwiegend Helferzellen), exprimieren CD4. Aufgaben von CD4⁺-Zellen im Rahmen der Immunantwort sind neben der Induktion der B-Zellfunktion die Aktivierung von Makrophagen, die Sekretion von Wachstums- und Differenzierungsfaktoren für lymphoide Zellen, die Sekretion von Faktoren, die nicht-lymphoide Zellfunktionen induzieren, die Induktion der Suppressor-, der NK- und der cytotoxischen T-Zellfunktion (Fauci, 1988).

Neben seiner wichtigen Rolle bei der Immunerkennung spielt CD4, ein Glykoprotein mit einem Molekulargewicht von 55.000, das außer auf T-Zellen auch in geringerem Ausmaß auf Monozyten/Makrophagen vorhanden ist, eine entscheidende Rolle bei der Infektion mit dem HIV-Virus, indem es als Rezeptor für das Virus fungiert. HIV ist der Erreger von AIDS ("acquired immunodeficiency syndrome"), einer schweren Erkrankung, die mit einer progressiven und irreversiblen Schädigung des Immunsystems einhergeht. Diese wird vor allem durch eine selektive Verringerung von CD4⁺-T-Zellen hervorgerufen.

Seit seiner Entdeckung wurde das HIV-Virus eingehend hinsichtlich seiner molekularen Biologie, seiner Infektiosität und der Mechanismen der Pathogenese untersucht.

HIV ist ein RNA-Retrovirus, das ursprünglich HTLV-III, LAV oder ARV bezeichnet wurde. Das früher als HIV bezeichnete Virus wird derzeit häufig auch als HIV-1 bezeichnet, um es von einem in westafrikanischen Patienten nachgewiesenen Virus (HIV-2) zu unterscheiden, das Verwandtschaft zum SIV-Virus aufweist und ein von AIDS nicht unterscheidbares Krankheitsbild verursacht.

Das HIV-Virusgenom ist gut charakterisiert. Es weist eine Länge von ca. 10 kb auf und umfaßt die flankierenden LTR ("long terminal repeat") Sequenzen, die regulatorische Sequenzen für die Replikation enthalten, sowie mindestens neun Gene. Diese Gene umfassen nicht nur die allen replikationsfähigen Retroviren gemeinsamen gag, pol und env-Gene, sondern auch Gene, die an der Reifung und Morphogenese beteiligt sind (vpu und vif), Gene, beteiligt an der Regulation der Virusreplikation (tat, rev und nef) und ein Gen unbekannter Funktion (vpr). Das tat-Gen spielt eine wichtige Rolle bei der Amplifikation der Virusreplikation, indem es für ein Protein mit trans-Aktivatorfunktion für die HIV-Genexpression codiert.

Nach seiner Bindung an das CD4-Molekül, das für HIV ein Rezeptor mit hoher Affinität ist (Dalgleish et al., 1984; Klatzmann et al., 1984; McDougal et al., 1986), wird das Virus in die Zelle aufgenommen und von seiner Hülle befreit. Zum Ablauf dieses Vorganges existieren widersprüchliche Ansichten. Es wurde u.a. vorgeschlagen, daß bei diesem Vorgang Rezeptorvermittelte Endozytose eine Rolle spielt (Maddon et al., 1986; Pauza und Price, 1988). Dagegen spricht jedoch die Beobachtung, daß für den Eintritt des Virus in die Zelle die Fusion des transmembranen Teils (gp41) der Virushülle mit der Zellmembran unabhängig vom pH-Wert erforderlich ist (Stein et al., 1987). Weiters wurde beobachtet, daß humanes CD4 exprimierende Mauszellen trotz Bindung des Virus an die Zelle nicht produktiv infiziert werden können. Dieses Ergebnis kann so interpretiert werden, daß außer CD4 gegebenenfalls noch andere Proteine auf humanen CD4⁺-Zellen für die Internalisierung des Virus mitverantwortlich sind.

Die Bindung des HIV-Virus an das CD4-Molekül erfolgt über das Virus-Hüllprotein (env).

Das Primärprodukt des env-Gens, gp160, ist ein Precursor, dessen Spaltung (während der Reifung auf dem Weg durch das ER und den Golgi-Apparat) die Virionproteine gp120 und gp41 ergibt. Die Spaltung von gp160 ist für die Fusion des Virus mit der Zelle und für die Infektiosität erforderlich. gp120 ist das äußere Hüll-Glycoprotein und auf der Außenseite der Membran infizierter Zellen und von Viruspartikeln vorhanden. Es weist keine Membran-Verankerungsdomäne auf und bleibt mit der Membran ausschließlich durch nichtkovalente Bindung mit gp41 verbunden.
gp120 enthält die für die Bindung des Virus an den Rezeptor wesentliche Determinante. Die hochaffine Bindung zwischen dem Virus und der Zellmembran wird durch Wechselwirkung zwischen einem Abschnitt von 40 Aminosäuren am C-Terminus von gp120 und einer Domäne nahe dem N-Terminus von CD4 vermittelt. Wenn auch zwischen den verschiedenen HIV-Stämmen erhebliche Unterschiede in den gp120 Sequenzen bestehen, sind die CD4-Bindungsdomänen zwischen HIV-1, HIV-2 und den verwandten SIV-Viren konserviert. Es wurden proteolytische Fragmente von 95 und 25 kDa isoliert, die offensichtlich domänenartige Unterteilungen von gp120 darstellen und an CD4 in gleicher Weise zu binden vermögen wie das ursprüngliche gp120 (Nygren et al., 1988).

Über den Ablauf des Fusionsvorganges gibt es verschiedene Theorien; unbestritten ist jedoch die Schlüsselrolle von gp41 für diesen Vorgang. gp41 weist eine hydrophobe Sequenz mit starker Homologie zu Fusionssequenzen am N-Terminus von Transmembran-Proteinen anderer Viren auf. Es wurde beobachtet, daß die env-Proteine ein Oligomeres bilden, wobei möglicherweise ein allosterisches Rearrangement des Oligomeren auf der Virusmembran das Einbringen des gp41-N-Terminus in die Ziel-Zellmembran und die Fusion fördert (derselbe Effekt wird für die Syncytienbildung zwischen infizierten Zellen verantwortlich gemacht).

Einer der als aussichtsreich angesehenen Ansätze, die HIV-Infektion zu blockieren, ist die Neutralisierung durch eine lösliche, sekretierte Form des CD4-Antigens (Smith et al.; 1987), die um die Bindung an gp120 konkurriert.

Eine therapeutische Möglichkeit, nach bereits erfolgter HIV-Infektion des Organismus die noch nicht infizierten Zellen zu schützen bzw. die Aktivierung des latenten Virus in den befallenden Zellen zu verhindern, besteht in der Verabreichung von die Virusreplikation inhibierenden Nukleinsäuremolekülen.

Für derartige therapeutische Anwendungen von Nukleinsäuren ist deren effiziente Aufnahme in die Zelle erforderlich.

Im Rahmen der vorliegenden Erfindung wurde überraschend festgestellt, daß Proteine, die an ein Zelloberflächenprotein binden, das von T-Zellen exprimiert wird, für den Transport von Nukleinsäuren in Zellen, die das Zelloberflächenprotein exprimieren, herangezogen werden können, wenn sie mit Polykationen konjugiert werden.

Es wurde festgestellt, daß der vom HIV-Virus bei der Infektion benutzte Rezeptor, CD4, für den Transport von Nukleinsäure in die Zelle ausgenutzt werden kann, indem die zu importierende Nukleinsäure mit einem Protein-Polykation-Konjugat komplexiert wird, dessen Proteinanteil ein Protein mit der Fähigkeit ist, an CD4 zu binden, und CD4 exprimierende Zellen mit den erhaltenen Protein-Polykation/DNA-Komplexen in Berührung gebracht werden.

Weiters konnte gezeigt werden, daß mit Hilfe von Antikörper-Polykation-Konjugaten, enthaltend einen Antikörper gegen CD7, DNA in Zellen der T-Zell-Abstammungslinie ("T-cell lineage") eingeführt und in diesen Zellen exprimiert wird. (CD7 ist ein Zelloberflächenprotein mit derzeit noch unbekannter physiologischer Rolle, das auf Thymocyten und reifen T-Zellen nachgewiesen wurde. CD7 ist ein verläßlicher Marker für die akute T-Zellenleukämie (Aruffo und Seed, 1987)).

Im Rahmen der vorliegenden Erfindung konnte somit anhand von Protein-Polykation-Konjugaten mit verschiedenen Proteinen, denen die Fähigkeit gemeinsam ist, an T-Zelloberflächenproteine zu binden, nachgewiesen werden, daß mit Hilfe solcher Konjugate in Zellen, die das jeweilige T-Zell-Oberflächenantigen exprimieren, Internalisierung und Expression von DNA erzielt werden kann.

Die Erfindung betrifft somit neue Protein-Polykation-Konjugate, die befähigt sind, mit Nukleinsäuren Komplexe zu bilden, wobei der Proteinanteil ein Protein mit der Fähigkeit ist, an ein Zelloberflächenprotein, das von T-Zellen exprimiert wird, zu binden, so daß die gebildeten Komplexe in Zellen, das Zelloberflächenprotein exprimieren, insbesondere T-Zellen, aufgenommen werden.

Im folgenden werden Proteine bzw. Fragmente davon mit der Fähigkeit, an Zelloberflächenproteine von T-Zellen zu binden, als T-Zell-bindende Proteine (TZBPs) bezeichnet.

Vertreter von Proteinen mit der Fähigkeit zur Bindung an CD4 (bzw. CD7) werden als "CD4 (CD7) bindendes Protein" oder "CD4BP (CD7BP)" bezeichnet.

Gegenstand der Erfindung sind weiters TZBP-Polykation/Nukleinsäure-Komplexe, in denen die erfindungsgemäßen Konjugate mit einer in die Zielzellen, die das T-Zelloberflächenantigen exprimieren, an das das TZBP bindet, zu transportierenden Nukleinsäure komplexiert ist.

Im Rahmen der vorliegenden Erfindung konnte gezeigt werden, daß DNA in Form der erfindungsgemäßen Komplexe effizient in Zellen, die das jeweilige T-Zelloberflächenantigen exprimieren, an das das TZBP bindet, aufgenommen und exprimiert wird, wobei die Aufnahme von DNA in die Zelle mit steigendem Konjugatgehalt zunahm.

Im Falle der Verwendung von Antikörpern als TZBPs kommen alle diejenigen, insbesondere monoklonalen, Antikörper gegen ein T-Zelloberflächenprotein bzw. Fragmente davon in Betracht, die an das jeweilige Zelloberflächenprotein binden, z.B. Fab'-Fragmente (Pelchen-Matthews et al., 1989).

Dazu zählen monoklonale Anti-CD4-Antikörper, die ein gp120-Epitop aufweisen, das mit dem Virus um die Bindung an dieses Epitop konkurriert.

Anstelle konventioneller monoklonaler Antikörper bzw. deren Fragmente können Antikörperabschnitte, bestehend aus einer Kombination von Segmenten der schweren und leichten Kette oder gegebenenfalls der schweren Kette allein, verwendet werden. Die Herstellung solcher "alternativer" Antikörper durch Klonierung mittels Polymerase-Kettenreaktion und Expression in E.coli wurde kürzlich beschrieben (Sastry et al., 1989; Orlandi et al., 1989; Chaudhary et al., 1990).

Als CD4BPs kommen weiters HIV-1-gp120 bzw. homologe Proteine verwandter Retroviren bzw. Fragmente davon in Betracht. Zur Verwendung im Rahmen der vorliegenden Erfindung geeignete gp120-Fragmente sind diejenigen, die zur Bindung an CD4 befähigt sind (Lasky et al., 1987), z.B die 95-kDa und 25-kDa-Fragmente, von denen nachgewiesen wurde, daß sie an CD4 binden (Nygren et al.; 1988). Solche Fragmente können z.B. erhalten werden, indem entweder zunächst das gesamte gp120 Protein auf rekombinantem Weg hergestellt und anschließend proteolytisch gespalten wird. Eine alternative Methode besteht in der rekombinanten Herstellung der Fragmente selbst.

Die Wahl des TZBP wird vor allem durch die Zielzellen bestimmt, z.B. durch bestimmte Oberflächenantigene oder Rezeptoren, die für einen Zelltyp spezifisch bzw. weitgehend spezifisch sind und somit einen gerichteten Import der Nukleinsäure in diesen Zelltyp ermöglichen.

Die erfindungsgemäßen Konjugate ermöglichen je nach Oberflächenantigen, an das das im Konjugat enthaltene Protein bindet, eine engere oder breitere Selektivität hinsichtlich der mit Nukleinsäure zu behandelnden, das T-Zelloberflächenprotein exprimierenden Zellen und somit einen flexiblen Einsatz therapeutisch oder gentherapeutisch wirksamer Nukleinsäure.

In Rahmen der vorliegenden Erfindung sind als Konjugat-Komponente TZBPs geeignet, die an die Zelle binden, wodurch die Konjugat/DNA-Komplexe, insbesondere über Endozytose, internalisiert werden, oder TZBPs, deren Bindung/Internalisierung über Fusion mit Zellmembranelementen erfolgt.

Wesentlich für die Eignung von TZBPs im Rahmen der vorliegenden Erfindung ist,
a) daß sie vom spezifischen Zelltyp, in den die Nukleinsäure eingebracht werden soll, erkannt werden und ihre Bindungsfähigkeit nicht oder nicht wesentlich beeinträchtigt wird, wenn sie mit dem Polykation konjugiert werden, und
b) daß sie im Rahmen dieser Eigenschaft in der Lage sind, Nukleinsäure auf dem von ihnen benutzten Weg in die Zelle "huckepack" mitzunehmen.

Unter der Voraussetzung, daß sie die unter a) und b) definierten Bedingungen erfüllen, sind grundsätzlich alle an T-Zelloberflächenantigene/Rezeptoren bindenden Proteine für die Anwendung im Rahmen der vorliegenden Erfindung geeignet. Dazu zählen Antikörper gegen T-Zelloberflächenproteine, die spezifisch auf einem oder mehreren Vertretern von Zellen eines bestimmten Differenzierungszustandes exprimiert werden, z.B. Antikörper gegen CD4, CD44, CD7, CD3, CD8 bzw. die entsprechenden Antikörperfragmente.

Für die gezielte Anwendung auf Tumorzellen kommen vor allem Antikörper gegen spezifisch auf T-Zellen exprimierte Zelloberflächenproteine, sog. Tumormarker, in Betracht, zB. CD7.

Neben Antikörpern und gp120(Fragmenten) sind im Rahmen der vorliegenden Erfindung alle natürliche Antigene, die die unter a) und b) angeführten Bedingungen erfüllen, geeignet.

Zu im Rahmen der vorliegenden Erfindung geeigneten Polykationen zählen homologe Polykationen, wie Polylysin, Polyarginin, Polyornithin, oder heterologe Polykationen mit zwei oder mehr unterschiedlichen positiv geladenen Aminosäuren, wobei diese Polykationen verschiedene Kettenlänge aufweisen können, weiters nichtpeptidische synthetische Polykationen wie Polyethylenimin. Geeignet sind weiters natürliche DNA bindende Proteine polykationischen Charakters wie Histone oder Protamine bzw. Analoge oder Fragmente davon.

Als Polykationen bzw. (poly)Peptide polykationischen Charakters können folgende Verbindungen verwendet werden:
a) Protamine: Dabei handelt es sich um kleine (MG bis ca. 8000), stark basische Proteine, deren positiv geladene Aminosäurereste (vor allem Arginine) für gewöhnlich in Gruppen angeordnet sind und die auf Grund ihres polykationischen Charakters die negativen Ladungen von Nukleinsäuren neutralisieren (Warrant et al., 1978). Die im Rahmen der vorliegenden Erfindung verwendbaren Protamine können natürlichen Ursprungs oder auf rekombinantem Weg hergestellt sein, wobei Mehrfachkopien hergestellt bzw. Modifikationen hinsichtlich Molekülgröße und Aminosäuresequenz vorgenommen werden können. Entsprechende Verbindungen können auch chemisch synthetisiert werden. Bei der Synthese eines künstlichen Protamins kann z.B. so vorgegangen werden, daß Aminosäurereste, die beim natürlichen Protamin Funktionen haben, die für die Transportfunktion unerwünscht sind (z.B. Kondensation von DNA) durch geeignete andere Aminosäuren ersetzt werden und/oder an einem Ende eine Aminosäure (z.B. Cystein) vorgesehen wird, die die gezielte Konjugation mit CD4BP ermöglicht.
b) Histone: Dies sind im Chromatin vorhandene kleine DNA-bindende Proteine mit einem hohen Anteil an positiv geladenen Aminosäuren (Lysin und Arginin), der sie befähigt, unabhängig von der Nukleotidsequenz an DNA zu binden und sie in Nukleosome zu falten, wozu besonders die argininreichen Histone H3 und H4 geeignet sind (Felsenfeld, 1978). Bezüglich der Herstellung und der Modifikationen gilt grundsätzlich das für Protamine Gesagte.
c) Synthetische Polypeptide, wie homologe Polypeptide (Polylysin, Polyarginin) oder heterologe Polypeptide (bestehend aus zwei oder mehr Vertretern positiv geladener Aminosäuren).
d) Nichtpeptische Kationen wie Polyethylenimine.

Die Größe der Polykationen wird bevorzugt so gewählt, daß die Summe der positiven Ladungen etwa 20 bis 500 beträgt, sie wird auf die zu transportierende Nukleinsäure abgestimmt.

Die erfindungsgemäßen TZBP-Polykation-Konjugate können auf chemischem Weg in für die Kopplung von Peptiden an sich bekannter Weise hergestellt werden, wobei, falls erforderlich, die Einzelkomponenten vor der Kopplungsreaktion mit Linkersubstanzen versehen werden (diese Maßnahme ist dann erforderlich, wenn von vornherein keine für die Kopplung geeignete funktionelle Gruppe, z.B. eine Mercapto- oder Alkoholgruppe, verfügbar ist. Bei den Linkersubstanzen handelt es sich um bifunktionelle Verbindungen, die zunächst mit funktionellen Gruppen der Einzelkomponenten zur Reaktion gebracht werden, worauf die Kopplung der modifizierten Einzelkomponenten durchgeführt wird.

Je nach gewünschten Eigenschaften der Konjugate, insbesondere im Hinblick auf deren Stabilität, kann die Kopplung erfolgen über
a) Disulfidbrücken, die unter reduzierenden Bedingungen wieder gespalten werden können (z.B. bei Verwendung von Succinimidylpyridyldithiopropionat (Jung et al., 1981).
b) Unter biologischen Bedingungen weitgehend stabile Verbindungen (z.B. Thioether durch Reaktion von Maleimido-Linkern mit Sulfhydrylgruppen des an die zweite Komponente gebundenen Linkers).
c) Unter biologischen Bedingungen labile Brücken, z.B. Esterbindungen, oder unter schwach sauren Bedingungen instabile Acetal- oder Ketalbindungen.

Es ist auch möglich, die erfindungsgemäßen Konjugate auf rekombinantem Weg herzustellen, was den Vorteil hat, genau definierte, einheitliche Verbindungen erhalten zu können, während bei der chemischen Kopplung Konjugat-Gemische entstehen, die aufgetrennt werden müssen.

Die rekombinante Herstellung der erfindungsgemäßen Konjugate ist nach für die Herstellung von chimären Polypeptiden bekannten Methoden durchführbar. Dabei können die polykationischen Peptide hinsichtlich ihrer Größe und Aminosäuresequenz variiert werden. Die gentechnologische Herstellung bietet auch den Vorteil, den TZBP-Anteil des Konjugats modifizieren zu können, indem z.B. durch geeignete Mutationen die Bindungsfähigkeit an das Zelloberflächenprotein gesteigert werden kann oder ein TZBP-Anteil, verkürzt auf den für die Bindung an das Zelloberflächenprotein maßgeblichen Teil des Moleküls, verwendet wird (z.B. Verwendung von gp120-Fragmenten oder "alternativer" Antikörper). Besonders zweckmäßig für die rekombinante Herstellung der erfindungsgemäßen Konjugate ist die Verwendung eines Vektors, der die für den TZBP-Anteil codierende Sequenz sowie einen Polylinker enthält, in den die jeweils benötigte für das polykationische Peptid codierende Sequenz eingesetzt wird. Auf diese Weise kann ein Satz von Expressionsplasmiden erhalten werden, von dem bei Bedarf das die gewünschte Sequenz enthaltende Plasmid zur Expression des erfindungsgemäßen Konjugats herangezogen wird.

Das molare Verhältnis TZBP:Polykation beträgt bevorzugt 10:1 bis 1:10, wobei zu berücksichtigen ist, daß es zur Ausbildung von Aggregaten kommen kann. Dieses Verhältnis kann jedoch bei Bedarf innerhalb weiterer Grenzen liegen, solange die Bedingung erfüllt ist, daß eine Komplexierung mit der bzw. den in die Zelle zu transportierenden Nukleinsäure(n) stattfindet und gewährleistet ist, daß der gebildete Komplex an das Zelloberflächenprotein gebunden und in die Zelle befördert wird. Dies kann durch einfach durchzuführende Versuche von Fall zu Fall überprüft werden, z.B. indem Zellinien, die das T-Zelloberflächenantigen exprimieren, mit den erfindungsgemäßen Komplexen in Berührung gebracht werden und daraufhin auf das Vorhandensein von Nukleinsäure, bzw. des Genprodukts, in der Zelle untersucht werden, z.B. durch Southern Blot Analyse, Hybridisierung mit radioaktiv markierten komplementären Nukleinsäuremolekülen, durch Amplifikation mit Hilfe der PCR oder durch Nachweis des Genproduktes eines Reportergens.

Das jeweils gewählte Verhältnis richtet sich vor allem nach der Größe des Polykationmoleküls sowie nach der Anzahl und Verteilung der positiv geladenen Gruppierungen, Kriterien, die auf Größe, Struktur sowie allenfalls vorhandene Modifikationen der zu transportierenden Nukleinsäure(n) abgestimmt werden. Die Polykationen können gleich oder verschieden sein.

Für bestimmte Anwendungsfälle bei Verwendung von Antikörpern als TZBPs, vor allem für das Screening zum Auffinden geeigneter Antikörper, kann es vorteilhaft sein, den Antikörper nicht direkt an das Polykation zu koppeln: für eine effiziente chemische Kopplung ist im allgemeinen eine größere Menge (mehr als 1 mg) Ausgangsantikörper erforderlich, außerdem wird durch die Kopplung gegebenenfalls die Antikörper-Bindungsdomäne inaktiviert. Um dieses Probleme zu umgehen und ein rasches Screening geeigneter Antikörper zu ermöglichen, kann zunächst ein Protein A-Polykation-Konjugat hergestellt werden, an das anschließend, gegebenenfalls in bereits mit Nukleinsäure komplexierter Form, unmittelbar vor der Transfektion der Zellen, der Antikörper über die F_{c}-Bindungsdomäne von Protein A gebunden wird (Surolia et al., 1982). Die mit den Protein A-Konjugaten gebildeten Nukleinsäure-Komplexe ermöglichen ein rasches Testen von Antikörpern auf ihre Eignung für den Import von Nukleinsäure in den jeweiligen zu behandelnden Zelltyp. Die Kopplung von Protein A mit dem jeweiligen Polykation erfolgt in analoger Weise wie die direkte Kopplung mit dem Antikörper. Bei der Anwendung von Protein-A-Antikörper-Polykation-Konjugaten kann es vorteilhaft sein, die zu behandelnden Zellen zuerst mit dem Antikörper zu inkubieren, die Zellen von überschüssigem Antikörper zu befreien und anschließend mit dem Protein A-Polykation/Nukleinsäure-Komplex zu behandeln. Die Herstellung von Protein A-Konjugaten kann je nach verwendetem Polykation auf rekombinantem Weg erfolgen.

Bei den in die Zelle zu transportierenden Nukleinsäuren kann es sich um DNAs oder RNAs handeln, wobei hinsichtlich der Nukleotidsequenz keine Beschränkungen bestehen. Unter "Nukleinsäuren" sind im Rahmen der vorliegenden Erfindung auch modifizierte Nukleinsäuren zu verstehen, sofern die Modifikation den polyanionischen Charakter der Nukleinsäuren und deren Komplexierung mit den erfindungsgemäßen Konjugaten nicht beeinträchtigt; zu diesen Modifikationen zählen z.B. die Substitution der Phosphodiestergruppe durch Phosphorothioate oder in der Verwendung von Nukleosidanalogen. Derartige Modifikationen sind dem Fachmann geläufig, eine Übersicht über Vertreter modifizierter Nukleinsäuren, die im allgemeinen als Nukleinsäureanaloga bezeichnet werden, sowie deren Wirkprinzip, sind in dem Artikel von Zon (1988) angegeben.

Bezüglich der Größe der Nukleinsäuren ermöglicht die Erfindung ebenfalls Anwendungen in einem weiten Bereich. Hinsichtlich der oberen Grenze besteht keine durch die erfindungsgemäßen Konjugate bedingte prinzipielle Limitierung, solange gewährleistet ist, daß die TZBP-Polykation/Nukleinsäurekomplexe in die Zelle befördert werden. Eine etwaige Begrenzung nach unten ergibt sich aus anwendungsspezifischen Gründen, weil z.B. Antisense-Oligonukleotide unter etwa 10 Nukleotiden auf Grund zu geringer Spezifität kaum für die Anwendung in Frage kommen. Mit Hilfe der erfindungsgemäßen Konjugate können auch Plasmide in die Zelle befördert werden.

Es ist auch möglich, gleichzeitig verschiedene Nukleinsäuren mit Hilfe der erfindungsgemäßen Konjugate in die Zelle zu befördern.

Beispiele für geeignete Nukleinsäuren sind die bereits angeführten Antisenseoligonukleotide oder Ribozyme mit virusinhibierender Wirkung aufgrund von Komplementarität zu für die Virusreplikation essentiellen Genabschnitten.

Bevorzugt als Nukleinsäureanteil der erfindungsgemäßen TZBP-Polykation-Nukleinsäurekomplexe mit inhibierender Wirkung aufgrund von Komplementarität ist Antisense-DNA, Antisense-RNA oder ein Ribozym bzw. das dafür codierende Gen. Bei der Anwendung von Ribozymen und Antisense-RNAs ist es besonders vorteilhaft, die dafür codierenden Gene, gegebenenfalls zusammen mit einem Carriergen, einzusetzen. Durch die Einführung des Gens in die Zelle wird gegenüber dem Import der RNA als solcher eine beträchtliche Amplifikation der RNA und damit ein für die angestrebte Hemmung der biologischen Reaktion ausreichender Vorrat gewährleistet. Besonders geeignete Carriergene sind die für die Transkription durch Polymerase III erforderlichen Transkriptionseinheiten, z.B. tRNA-Gene. In diese können z.B. Ribozymgene derart eingefügt werden, daß bei Transkription das Ribozym Teil eines kompakten Polymerase III-Transkripts ist. Geeignete genetische Einheiten, enthaltend ein Ribozymgen und ein von Polymerase III transkribiertes Carriergen, sind in der Europäischen Patentanmeldung A1 0 387 775 geoffenbart. Mit Hilfe des Transportsystems gemäß der vorliegenden Erfindung kann die Wirkung dieser genetischen Einheiten verstärkt werden, indem eine erhöhte Ausgangskonzentration des Gens in der Zelle gewährleistet wird.

Als Zielsequenzen für die Konstruktion komplementärer Antisenseoligonukleotide oder Ribozyme bzw. der dafür codierenden Gene, die bei der Therapie von AIDS zur Anwendung kommen können, sind grundsätzlich sämtliche Sequenzen des HIV-Gens geeignet, deren Blockierung die Inhibierung der viralen Replikation und Expression zur Folge hat. In erster Linie in Frage kommende Zielsequenzen sind Sequenzen mit regulatorischer Funktion, vor allem des tat-, rev- oder nef-Gens. Geeignete Sequenzen sind weiters die Initiations-, Polyadenylierungs-, Splicing tRNA Primer-Bindungsstelle (PBS) der LTR-Sequenz oder die tar-Sequenz.

Außer Nukleinsäuremolekülen, die aufgrund ihrer Komplementarität zu viralen Genen inhibieren, können auch Gene mit anderem Wirkmechanismus eingesetzt werden, z.B. solche, die für Virusproteine, enthaltend sog. trans-dominante Mutationen, codieren (Herskowitz, 1987). Die Expression der Genprodukte in der Zelle resultiert in Proteinen, die in ihrer Funktion das entsprechende Wildtyp-Virusprotein dominieren, womit dieses seine für die Virusreplikation normalerweise geleistete Aufgabe nicht erfüllen kann und die Virusreplikation wirksam inhibiert wird. Geeignet sind grundsätzlich trans-dominante Mutanten von Virusproteinen, die für die Replikation und Expression erforderlich sind, z.B. Gag-, Tat- und Rev-Mutanten, von denen inhibierende Wirkung auf die HIV-Replikation nachgewiesen wurde (Trono et al., 1989; Green et al., 1989; Malim et al., 1989).

Vertreter therapeutisch wirksamer Nukleinsäuren sind weiters solche mit inhibierender Wirkung auf Oncogene.

Mit Hilfe der vorliegenden Erfindung können auch Gene bzw. Abschnitte davon in die Zelle transportiert werden, deren Expressionsprodukte eine Funktion bei der Signalübertragung haben, um auf diese Weise die Signalübertragung in den Zielzellen positiv zu beeinflussen und damit z.B. die Überlebensfähigkeit von T-Zellen zu steigern.

Als Zielzellen für die Immuntherapie kommen in erster Linie T-Zellen vom Typ der sog. "Killerzellen", die zytotoxische Aktivität aufweisen und auch als "TIL's" ("tumour infiltrating lymphocytes") bezeichnet werden, in Betracht. Die erfindungsgemäßen Konjugate können als Alternative zum Gentransfer mittels retroviraler Vektoren verwendet werden, um in diese Zellen DNA einzubringen. Die DNA enthält vorzugsweise ein Gen, das für ein Protein mit der Fähigkeit codiert, die zytotoxische Aktivität dieser Zellen zu erhöhen, z.B. TNF oder IFN-α. Die in Killerzellen eingebrachte DNA kann auch das IL-2-Gen enthalten, um durch die Expression von IL-2 die Proliferation der Zellen lokal zu verstärken.

Prinzipiell sind sämtliche Gene bzw. Genabschnitte im Rahmen der vorliegenden Erfindung geeignet, die in ein T-Zelloberflächenprotein exprimierenden Zellen einen therapeutischen oder gentherapeutischen Effekt haben.

Das Verhältnis Nukleinsäure: Konjugat kann innerhalb eines weiten Bereichs schwanken, wobei es nicht unbedingt erforderlich sein muß, sämtliche Ladungen der Nukleinsäure zu neutralisieren. Dieses Verhältnis wird von Fall zu Fall nach Kriterien wie Größe und Struktur der zu transportierenden Nukleinsäure, Größe des Polykations, Anzahl und Verteilung seiner Ladungen, derart einzustellen sein, daß ein für die jeweilige Anwendung günstiges Verhältnis zwischen Transportfähigkeit und biologischer Aktivität der Nukleinsäure besteht. Dieses Verhältnis kann zunächst grob eingestellt werden, etwa an Hand der Verzögerung der Wanderungsgeschwindigkeit der DNA in einem Gel (z.B. mittels "Mobility Shift" auf einem Agarosegel) oder durch Dichtegradientenzentrifugation. Nach Erhalt dieses vorläufigen Verhältnisses kann es zweckmäßig sein, im Hinblick auf die in der Zelle maximal verfügbare Aktivität der Nukleinsäure Transportversuche mit dem radioaktiv markierten Komplex durchzuführen und den Konjugat-Anteil gegebenenfalls derart zu reduzieren, daß die verbleibenden negativen Ladungen der Nukleinsäure dem Transport in die Zelle nicht hinderlich sind.

Die Herstellung der TZBP-Polykation/Nukleinsäure-Komplexe kann nach in an sich für die Komplexierung polyionischer Verbindungen bekannten Methoden vorgenommen wurden. Eine Möglichkeit, unkontrollierte Aggregation bzw. Ausfällung zu vermeiden, besteht darin, die beiden Komponenten bei hoher Verdünnung (≦50 »g/ml) zu mischen.

Die über Endozytose in höhere eukaryotische Zellen aufnehmbaren TZBP-Polykation-Nukleinsäure-Komplexe können zusätzlich eine oder mehrere Polykationen in nicht-kovalent gebundener Form, die gegebenenfalls identisch sind mit dem Polykation im Konjugat, derart enthalten, daß die durch das Konjugat erzielte Internalisierung und/oder Expression der Nukleinsäure gesteigert wird.

Mit Hilfe derartiger Maßnahmen, die Gegenstand der Deutschen Patentanmeldung DE-A1-41 04 186.0 sind, wird bei zumindest gleicher Transfektions/Expressions-Effizienz eine geringere Menge, bezogen auf die in die Zelle zu importierende Nukleinsäure-Menge, TZBP-Polykation-Konjugat benötigt, was einerseits einen geringeren Synthese-Aufwand bedeutet. Eine geringere Konjugatmenge kann andererseits dann von Vorteil sein, wenn der Effekt vermieden werden soll, daß durch eine größere Anzahl von TZBP-Molekülen innerhalb eines Komplexes gleich mehrere benachbarte "Andock-Stellen" besetzt werden und in der Folge für weitere Komplexe nicht mehr verfügbar sein können. Die Anzahl der in den Komplexen enthaltenen Menge an TZBP auf das erforderliche Minimum zu beschränken, d.h. die Konjugatmenge möglichst gering zu halten und mit freiem Polykation zu verdünnen, ist insbesondere auch dann von Vorteil, wenn die Zahl von Zelloberflächenproteinen auf den zu behandelnden Zielzellen gering ist.

Mit Hilfe solcher Maßnahmen kann die Leistungsfähigkeit von Konjugaten, die für sich weniger effizient sind, beträchtlich erhöht werden und die Leistungsfähigkeit von an sich schon sehr effizienten Konjugaten noch weiter gesteigert werden.

Hinsichtlich der qualitativen Zusammensetzung der erfindungsgemäßen Komplexe werden im allgemeinen zunächst die in die Zelle zu importierende Nukleinsäure und das TZBP festgelegt. Die Nukleinsäure wird vor allem durch den in der Zelle zu erzielenden biologischen Effekt definiert, z.B. durch die Zielsequenz des zu inhibierenden oder - im Falle der Anwendung im Rahmen der Gentherapie - des zur Expression zu bringenden Gens bzw. Genabschnitts, z.B. zwecks Substitution eines defekten Gens. Gegebenenfalls ist die Nukleinsäure modifiziert, bedingt z.B. durch eine für den jeweiligen Anwendungsfall erforderliche Stabilität.

Ausgehend von der Festlegung von Nukleinsäure und TZBP wird das Polykation auf diese Parameter abgestimmt, wobei die Größe der Nukleinsäure, vor allem im Hinblick auf eine weitgehende Neutralisierung der negativen Ladungen, eine wesentliche Rolle spielt.

Für die Wahl der gegebenenfalls in den Komplexen enthaltenen nicht-kovalent gebundenen Polykationen ist entscheidend, daß durch ihren Zusatz gegenüber der durch die Konjugate erzielbaren Internalisierung/Expression der Nukleinsäure eine Steigerung bewirkt wird.

Ebenso wie die qualitative Zusammensetzung richtet sich auch die quantitative Zusammensetzung der Komplexe nach mehreren Kriterien, die miteinander in funktionellem Zusammenhang stehen. Für die Entscheidung, nichtkonvalent gebundenes Polykation als Komplex-Bestandteil vorzusehen, ist u.a. maßgeblich, ob und in welchem Ausmaß es erforderlich oder erwünscht ist, die Nukleinsäure zu kondensieren, welche Ladung der Gesamtkomplex aufweisen soll, in welchem Ausmaß Bindungs- und Internalisierungsfähigkeit für den jeweiligen Zelltyp gegeben ist und in welchem Ausmaß deren Steigerung erwünscht bzw. erforderlich ist. Weitere Parameter für die Zusammensetzung des Komplexes sind die Zugänglichkeit des TZBPs für das Zelloberflächenprotein, wobei dafür die Art maßgeblich ist, wie dieses Protein innerhalb des Komplexes der Zelle gegenüber präsentiert wird. Wesentlich ist weiters die Zugänglichkeit der Nukleinsäure in der Zelle, um deren bestimmungsgemäße Funktion auszuüben.

Die in nicht kovalent-gebundener Form in den Komplexen gegebenenfalls enthaltenen Polykationen können gleich oder verschieden sein von den im Konjugat enthaltenen. Ein wesentliches Kriterium für deren Auswahl ist die Größe der Nukleinsäure, insbesondere im Hinblick auf deren Kondensierung; bei kleineren Nukleinsäure-Molekülen ist im allgemeinen eine Kompaktierung nicht erforderlich. Die Auswahl der Polykationen nach Art und Menge wird außerdem auf das Konjugat abgestimmt, wobei vor allem das im Konjugat enthaltene Polykation zu berücksichtigen ist: ist das Polykation z.B. eine Substanz, die keine oder nur geringe Fähigkeit zur DNA-Kondensation aufweist, ist es im Hinblick auf eine effiziente Internalisierung der Komplexe im allgemeinen zweckmäßig, solche Polykationen einzusetzen, die diese Eigenschaft in stärker ausgeprägtem Maß besitzen. Ist der das im Konjugat enthaltene Polykation selbst eine Nukleinsäure kondensierende Substanz und wird bereits eine im Hinblick auf effiziente Internalisierung ausreichende Kompaktierung der Nukleinsäure bewirkt, wird zweckmäßigerweise ein Polykation verwendet, das aufgrund anderer Mechanismen eine Steigerung der Expression bewirkt.

Wesentlich für das gegebenenfalls im Komplex zusätzlich enthaltene nicht-kovalent gebundene Polykation ist seine Fähigkeit, Nukleinsäure zu kondensieren und/oder diese vor unerwünschtem Abbau in der Zelle zu schützen.

Gegenstand der Erfindung ist weiters ein Verfahren zum Einführen von Nukleinsäure(n) in menschliche oder tierische Zellen, die das T-Zelloberflächenantigen exprimieren, wobei bevorzugt ein unter physiologischen Bedingungen löslicher TZBP-Polykation/Nukleinsäure-Komplex mit den Zellen, insbesondere T-Zellen, in Berührung gebracht wird.

Im Rahmen der vorliegenden Erfindung wurde als DNA-Komponente das Luciferase-Gen als Reporter-Gen verwendet. (In Vorversuchen mit Transferrin-Polykation/DNA-Komplexen, in denen das Luciferase-Gen als Reportergen verwendet wurde, hatte sich gezeigt, daß aus der Effizienz des Imports des Luciferase-Gens auf die Anwendbarkeit anderer Nukleinsäuren geschlossen werden kann; die verwendete Nukleinsäure ist in qualitativer Hinsicht kein limitierender Faktor für die Anwendung von Protein-Polykation-DNA-Komplexen.)

Es kann für bestimmte Ausführungsformen der vorliegenden Erfindung vorteilhaft sein, Bedingungen zu schaffen, unter denen der Abbau der in die Zelle importierten Nukleinsäure verringert oder ausgeschaltet wird.

Bedingungen, unter denen der Abbau von Nukleinsäuren inhibiert wird, können durch Zusatz sog. lysosomatroper Substanzen geschaffen werden. Von diesen Substanzen ist bekannt, daß sie die Aktivität von Proteasen und Nukleasen in Lysosomen hemmen und somit den Abbau von Nukleinsäuren verhindern können (Luthmann u. Magnusson, 1983).

Zu diesen Substanzen zählen Chloroquin, Monensin, Nigericin, Ammoniumchlorid und Methylamin.

Das Erfordernis für die Anwendung einer Substanz aus der Gruppe der lysosomatropen Substanzen im Rahmen der vorliegenden Erfindung richtet sich u.a. nach dem zu behandelnden Zelltyp oder bei Verwendung unterschiedlicher Antikörper gegebenenfalls nach unterschiedlichen Mechanismen, über die die Aufnahme der Komplexe in die Zelle erfolgt. So wurde z.B. im Rahmen der vorliegenden Erfindung eine unterschiedliche Beeinflussung des DNA-Imports in die Zelle durch Chloroquin bei Verwendung unterschiedlicher Antikörper (monoklonale anti-CD4-Antikörper) festgestellt.

Es ist in jedem Fall notwendig, das Erfordernis bzw. die Eignung derartiger Substanzen im Rahmen der vorliegenden Erfindung in Vorversuchen zu testen.

Gegenstand der Erfindung sind weiters pharmazeutische Zubereitungen, enthaltend als wirksame Komponente eine oder mehrere therapeutisch oder gentherapeutisch wirksame Nukleinsäure(n), komplexiert mit einem TZBP-Polykation-Konjugat (TZBP-Polykation-Konjugat und Nukleinsäure können auch getrennt vorliegen und unmittelbar vor der therapeutischen Anwendung komplexiert werden).

Als therapeutisch wirksame Nukleinsäuren kommen die bereits angeführten Antisenseoligonukleotide oder Ribozyme bzw. die dafür codierenden Gene oder für trans-dominante Mutanten codierende Gene in Betracht, die inhibierende Wirkung auf in den jeweiligen Zielzellen enthaltene endogene oder exogene Gene oder Genprodukte haben. Darunter sind z.B. diejenigen Gene zu verstehen, die aufgrund ihrer Sequenzspezifität (Komplementarität zu Zielsequenzen, Codierung für trans-dominante Mutanten (Herskowitz, 1987)) eine intrazelluläre Immunität (Baltimore, 1988) gegen HIV bewirken und bei der Therapie des AIDS-Syndroms oder zur Verhinderung der Aktivierung des Virus nach der Infektion verwendet werden können.

Die pharmazeutischen Zubereitungen können verwendet werden, um im menschlichen oder tierischen Organismus virale Sequenzen, z.B. HIV oder verwandte Retroviren zu inhibieren. Ein Beispiel für die therapeutische Anwendung durch Inhibierung eines verwandten Retrovirus ist die Behandlung der proliferativen T-Zelleukämie, die durch das HTLV-1 Virus hervorgerufen wird.

Neben der Behandlung viraler T-Zelleukämien kann die vorliegende Erfindung auch für die Therapie nichtviraler Leukämien verwendet werden. Die Beteiligung von Oncogenen (abl, bcr, ras, rat, c-myc, N-myc) an der Entstehung lymphatischer Leukämien wurde in jüngster Zeit nachgewiesen; die Existenz weiterer Oncogene wird aufgrund von beobachteten spezifischen Chromosomentranslokationen für wahrscheinlich erachtet. Die Klonierung wird somit Kenntnis der DNA-Sequenz dieser Oncogene bietet die Grundlage für die Konstruktion Oncogen-inhibierender Nukleinsäuremoleküle und damit für eine weitere therapeutische Einsatzmöglichkeit der vorliegenden Erfindung.

Ein weiteres wichtiges Anwendungsgebiet ist die Gentherapie. Prinzipiell können im Rahmen der Gentherapie mit Hilfe der vorliegenden Erfindung all diejenigen Gene bzw. Abschnitte davon in ein T-Zelloberflächenprotein exprimierende Zielzellen eingeführt werden, deren Expression in diesem Zelltyp einen therapeutischen Effekt erzielt, z.B. durch Substitution genetisch bedingter Defekte oder Auslösen einer Immunantwort.

Obwohl das Schwergewicht für die Anwendung der Erfindung auf Vertreter von Zellen der T-Lymphozyten-Abstammungslinie gelegen ist, kann sie auch auf Vertreter anderer Zellspezies, sofern diese Zellen das T-Zelloberflächenprotein exprimieren, angewendet werden.

### Figurenübersicht

- Fig.1:: Import von anti-CD4-Polylysin/pRSVL-Komplexen in CD4⁺-CHO-Zellen
- Fig.2:: Import von anti-CD4-Polylysin/pRSVL-Komplexen in CD4⁺-CHO-Zellen
- Fig.3:: Import von gp120-Polylysin/pRSVL-Komplexen in CD4⁺ CHO-Zellen
- Fig.4:: Import von gp120-Polylysin190/pRSVL-Komplexen, enthaltend nicht-kovalent gebundenes Poly(D)Lysin240, in CD4⁺ CHO-Zellen
- Fig.5:: Import von gp120-Polylysin120/pRSVL-Komplexen in CD4⁺ HeLa-Zellen
- Fig.6:: Transfer und Expression von DNA in H9-Zellen mittels antiCD7-Polylysin190-Konjugaten
- Fig.7:: Transfektion von CD4⁺-Zellen mit Protein A-Polylysin-Konjugaten

Die Erfindung wird anhand der nachfolgenden Beispiele illustriert.

### Beispiel 1

### Herstellung von antiCD4-Polylysin90-Konjugaten

Die Kopplung erfolgte analog literaturbekannter Methoden durch Einführung von Disulfidbrücken nach Modifizierung mit Succinimidyl-Pyridyldithio-propionat (SPDP, Jung et al., 1981).

Eine Lösung von 1,7 mg antiCD4-Antikörper (OKT4A, Ortho Diagnostic Systems) in 50 mM Natriumphosphatpuffer pH 7,8 wurde mit 11 »l 10 mM ethanolischer Lösung von SPDP (Pharmacia) versetzt.

Nach 1 h bei Raumtemperatur wurde über eine Gelsäule Sephadex G 25 filtriert (Eluens 100 mM HEPES-Puffer pH 7,3), wobei 1,4 mg antiCD4, modifiziert mit 75 nmol Pyridyldithiopropionatresten, erhalten wurden. Poly(L)lysin90 (durchschnittlicher Polymerisationsgrad von 90 Lysinresten (Sigma), Fluoreszenzmarkierung mittels FITC) wurde analog mit SPDP modifiziert und durch Behandlung mit Dithiothreitol und nachfolgender Gelfiltration in die mit freien Mercaptogruppen modifizierte Form gebracht.
Eine Lösung von 38 nmol Polylysin90, modifiziert mit 120 nmol Mercaptogruppen, in 0,5 ml 20 mM Natriumacetatpuffer, wurde unter Sauerstoffausschluß mit oben angeführtem modifiziertem antiCD4 gemischt und über Nacht bei Raumtemperatur stehen gelassen. Die Isolierung der Konjugate erfolgte durch Gelpermeations-Chromatographie (Superose 12, 500 mM Guanidiniumhydrochlorid, pH 7,3); nach Dialyse gegen 25 mM HEPES pH 7,3 wurden entsprechende Konjugate, bestehend aus 1,1 mg anticD4-Antikörper, modifiziert mit 11 nmol Polylysin90, erhalten.

### Beispiel 2

### Herstellung von antiCD4-Polylysin190-Konjugaten

Eine Lösung von 1,0 mg (6.25nmol) antiCD4-Antikörper (OKT4A, Ortho Diagnostic Systems) in 0,3 ml 50 mM HEPES pH 7,8 wurde mit 37 »l 1 mM ethanolischer Lösung von Succinimidyl-Pyridyldithio-propionat (SPDP,Pharmacia) versetzt. Nach 1 h bei Raumtemperatur wurde über eine Gelsäule Sephadex G 25 filtriert (Eluens 100 mM HEPES-Puffer pH 7,9), wobei 0,85 mg (5,3 nmol) antiCD4, modifiziert mit 30 nmol Pyridyldithiopropionatresten, erhalten wurden. Poly(L)lysin190 (durchschnittlicher Polymerisationsgrad von 190 Lysinresten (Sigma), Fluoreszenzmarkierung mittels FITC) wurde analog mit SPDP modifiziert und durch Behandlung mit Dithiothreitol und nachfolgender Gelfiltration in die mit freien Mercaptogruppen modifizierte Form gebracht. Eine Lösung von 7,7 nmol Polylysin190, modifiziert mit 25 nmol Mercaptogruppen, in 0,13 ml 30 mM Natriumacetatpuffer wurde unter Sauerstoffausschluß mit oben angeführtem modifiziertem antiCD4 (in 0,5 ml 300mM HEPES pH 7,9) gemischt und über Nacht bei Raumtemperatur stehen gelassen. Das Reaktionsgemisch wurde durch Zugabe von 5 M NaCl auf einen Gehalt von ungefähr 0,6 M gebracht. Die Isolierung der Konjugate erfolgte durch Ionenaustauschchromatographie (Mono S, Pharmacia, 50 mM HEPES pH 7,3, Salzgradient 0,6 M bis 3 M NaCl); nach Dialyse gegen 10 mM HEPES pH 7,3 wurden entsprechende Konjugate, bestehend aus 0,35 mg (2,2 nmol) antiCD4-Antikörper, modifiziert mit 3,9 nmol Polylysin190, erhalten.

### Beispiel 3

### Herstellung von gp120-Polylysin190-Konjugaten

Die Koppelung erfolgte analog literaturbekannter Methoden entweder durch Einführung von Disulfidbrücken nach Modifizierung mit Succinimidyl-Pyridyldithiopropionat oder durch Thioether-Verknüpfung nach Modifizierung mit 6-Maleimidocapronsäure-N-hydroxysuccinimidester (EMCS, Sigma) (Fujiwara et al., 1981).

### a) Disulfidverknüpfte gp120-Polylysin190-Konjugate:

Eine Lösung von 3 mg rekombinantem gp120 (hergestellt nach der von Lasky et al., 1986, beschriebenen Methode) in 50 mM HEPES pH 7,8 wurde mit 7 »l 10 mM ethanolischer Lösung von SPDP versetzt. Nach 1 h bei Raumtemperatur wurde über eine Gelsäule Sephadex G 25 filtriert (Eluens 100 mM HEPES-Puffer pH 7,9) und man erhielt dabei 2,8 mg (23 nmol) rgp120, modifiziert mit 67 nmol Pyridyldithiopropionatresten.
Eine Lösung von 6,6 nmol Polylysin190, fluoreszenzmarkiert und, wie oben für die antiCD4-Konjugate beschrieben, modifiziert mit 23 nmol Mercaptogruppen, in 120 »l 30 mM Natriumacetat wurde unter Sauerstoffausschluß mit dem modifizierten rgp120 gemischt und über Nacht bei Raumtemperatur stehen gelassen. Das Reaktionsgemisch wurde durch Zugabe von 5M NaCl auf einen Gehalt von ungefähr 0,6 M gebracht. Die Isolierung der Konjugate erfolgte durch Ionenaustauschchromatographie (Mono S, Pharmacia, 50mM HEPES pH 7,3, Salzgradient 0,6 M bis 3 M NaCl); nach Fraktionierung und Dialyse gegen 25 mM HEPES pH 7,3 wurden zwei Konjugatfraktionen A und B, bestehend aus 0,33 mg rgp120 modifiziert mit 1,3 nmol Polylysin190 (im Falle der Fraktion A), bzw. 0,34 mg rgp120 modifiziert mit 3,2 nmol Polylysin190 (Fraktion B) erhalten.

### b) Thioether-verknüpfte gp120-Polylysin190-Konjugate:

Eine Lösung von 2 mg rekombinantem gp120 (rgp120) in 0,45 ml 100 mM HEPES pH 7,9 wurde mit 17 »l einer 10 mM Lösung von EMCS in Dimethylformamid versetzt. Nach 1 h bei Raumtemperatur wurde über eine Gelsäule Sephadex G 25 filtriert (Eluens 100 mM HEPES-Puffer 7,9). Die Produktlösung (1,2 ml) wurde sogleich unter Sauerstoffausschluß umgesetzt mit einer Lösung von 9,3 nmol Polylysin190, fluoreszenzmarkiert und wie oben (antiCD4-Konjugate) beschrieben modifiziert mit 30 nmol Mercaptogruppen (in 90 »l 30 mM Natriumacetat pH 5,0), und über Nacht bei Raumtemperatur stehen gelassen. Das Reaktionsgemisch wurde durch Zugabe von 5M NaCl auf einen Gehalt von ca. 0,6 M gebracht. Die Isolierung der Konjugate erfolgte durch Ionenaustauschchromatographie (Mono S, Pharmacia, 50mM HEPES pH 7,3, Salzgradient 0,6 M bis 3 M NaCl); nach Fraktionierung und Dialyse gegen 25 mM HEPES pH 7,3 wurden drei Konjugatfraktionen A, B und C erhalten, bestehend aus 0,40 mg rgp120 modifiziert mit 1,9 nmol Polylysin190 (im Falle der Fraktion A), bzw. 0,25 mg rgp 120 modifiziert mit 2,5 nmol Polylysin190 (Fraktion B), bzw. 0,1 mg rgp 120 modifiziert mit 1,6 nmol Polylysin190 (Fraktion C).

### Beispiel 4

### Herstellung von antiCD7-Polylysin190-Konjugaten

Eine Lösung von 1,3 mg antiCD7-Antikörper (Immunotech) in 50 mM HEPES pH 7,9 wurde mit 49 »l 1 mM ethanolischer Lösung von SPDP (Pharmacia) versetzt. Nach 1 h bei Raumtemperatur wurde über eine Gelsäule Sephadex G 25 filtriert (Eluens 50 mM HEPES-Puffer pH 7,9) wobei 1,19 mg (7,5 nmol) antiCD7, modifiziert mit 33 nmol Pyridyldithiopropionatresten, erhalten wurden. Poly(L)lysin190, Fluoreszenzmarkierung mittels FITC, wurde analog mit SPDP modifiziert und durch Behandlung mit Dithiothreitol und nachfolgender Gelfiltration in die mit freien Mercaptogruppen modifizierte Form gebracht.

Eine Lösung von 11 nmol Polylysin190, modifiziert mit 35 nmol Mercaptogruppen, in 0,2 ml 30 mM Natriumacetatpuffer wurde unter Sauerstoffausschluß mit oben angeführtem modifiziertem antiCD7 (in 0,5 ml 300mM HEPES pH 7,9) gemischt und über Nacht bei Raumtemperatur stehen gelassen. Das Reaktionsgemisch wurde durch Zugabe von 5 M NaCl auf einen Gehalt von ungefähr 0,6 M gebracht. Die Isolierung der Konjugate erfolgte durch Ionenaustauschchromatographie (Mono S, Pharmacia, 50 mM HEPES pH 7,3, Salzgradient 0,6 M bis 3 M NaCl); nach Dialyse gegen 10 mM HEPES pH 7,3 wurden entsprechende Konjugate, bestehend aus 0,51 mg (3,2 nmol) antiCD7-Antikörper, modifiziert mit 6,2 nmol Polylysin190, erhalten.

### Beispiel 5

### Herstellung von Komplexen von Antikörper-Polykation-Konjugaten mit DNA

Die Komplexe wurden hergestellt, indem verdünnte Lösungen von DNA (30 »g/ml oder weniger in 150 mM NaCl, 20 mM HEPES pH 7,3) mit den in den Beispielen 1, 2 und 4 erhaltenen Antikörper-Polylysin-Konjugaten (100 »g/ml oder weniger) vermischt wurden. Als DNA wurde pRSVL Plasmid-DNA (De Wet et al., 1987) verwendet, hergestellt mittels Triton-X Lyse Standard-Methode (Maniatis, 1982), gefolgt von CsCl/EtBr Gleichgewichtsdichtegradienten-Zentrifugation, Entfärbung mit Butanol-1 und Dialyse gegen 10 mM Tris/HCl pH 7,5, 1 mM EDTA.
Um ein Ausfällen der DNA-Komplexe zu verhindern, wurde phosphatfreier Puffer verwendet (Phosphate verringern die Löslichkeit der Konjugate).

### Beispiel 6

### Transfer und Expression von DNA in CD4⁺ CHO-Zellen mittels antiCD4-Polylysin90-Konjugaten

In diesem und den folgenden Beispielen wurde Plasmid-DNA, enthaltend das Photinus pyralis-Luciferase-Gen als Reporter-Gen, verwendet, um Gentransfer und Expression zu untersuchen. In den Figuren, die die Ergebnisse der Versuche darstellen, beziehen sich die dargestellten Werte für die Luciferase-Aktivität auf die Aktivität der gesamten Zellprobe.

CD4⁺ CHO-Zellen (Lasky et al., 1987) wurden zu 5x10⁵ Zellen pro T-25-Flasche in Ham's F-12-Medium (Ham, 1965) plus 10 % FCS (fötales Kälberserum) ausgesät.
18 h später wurden die Zellen zweimal mit Ham's F-12-Medium ohne Serum gewaschen und in diesem Medium (5 ml) 5 h lang bei 37°C bebrütet.

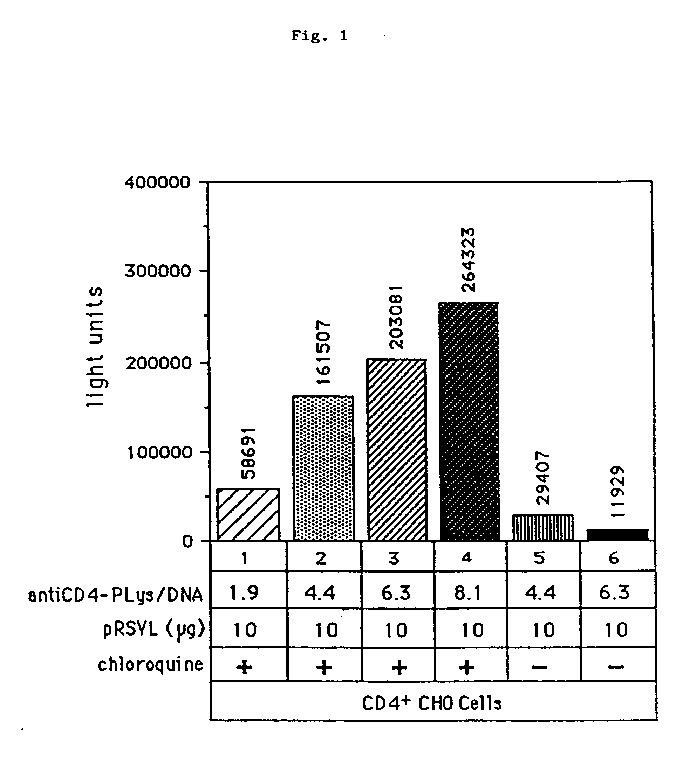

Anti-CD4-Polylysin/pRSVL Komplexe wurden bei Endkonzentrationen von DNA von 10 »g/500 »l in 150 mM NaCl, 20 mM HEPES pH 7,5 hergestellt, wie in Beispiel 5 beschrieben. Anti-CD4-Polylysin90 (8,4 nmol Polylysin90/mg anti-CD4) wurde in den angegebenen Masse-Verhältnissen (von 1,9 bis 8,1, ausgedrückt als Masse von anti-CD4) eingesetzt. In den Proben 1 - 4 wurden die Komplexe den Zellen in Ham's F-12-Medium ohne Serum, enthaltend 100 »M Chloroquin zugefügt; in den Proben 5 und 6 wurde Chloroquin weggelassen. Nach einer 4stündigen Inkubation wurden die Zellen zweimal mit Medium plus 10 % FCS gewaschen und in diesem Medium inkubiert. In den Proben 5 und 6 wurde dasselbe Volumen an Serum enthaltendem Medium zu den Zellen gefügt. Nach 20 h wurden alle Zellen mit frischem, Serum enthaltendem Medium gewaschen und 48 h später geerntet. Aliquots von Extrakten (ca. 1/5 jeder Probe), entsprechend gleicher Proteinmenge, wurden auf Luciferase-Aktivität untersucht (De Wet et al., 1987). Die Bioluminiszenz wurde unter Verwendung des Clinilumats (Berthold, Wildbach, BRD) gemessen. Das Ergebnis dieser Untersuchungen ist in Fig.1 dargestellt. Es zeigte sich, daß DNA mit Hilfe der erfindungsgemäßen Konjugate in CD4⁺-Zellen importiert wird und die importierte DNA exprimiert wird, wobei die Effizienz des DNA-Imports proportional dem anti-CD4/Polylysin-Gehalt ist.

### Beispiel 7

### Transfer und Expression von DNA in CD4⁺ CHO-Zellen mittels antiCD4-Polylysin190-Konjugaten

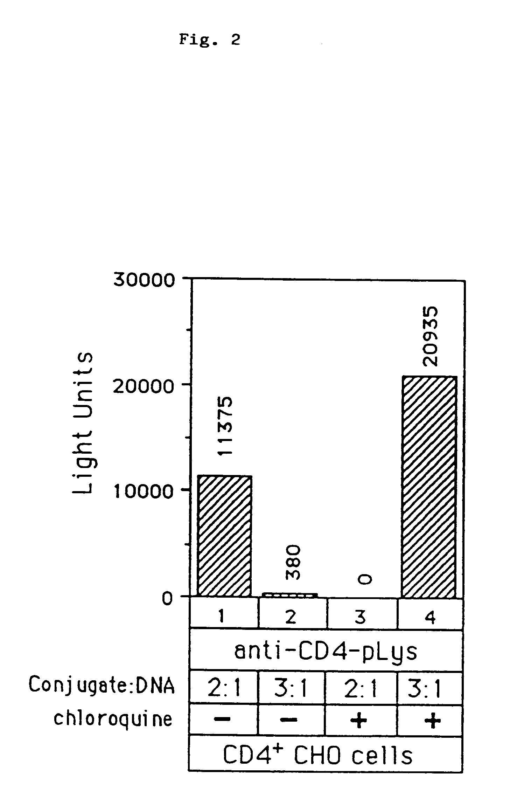

Zunächst wurden CD4⁺ CHO-Zellen gezüchtet, wie in Beispiel 6 angegeben. Konjugat/DNA -Komplexe, hergestellt wie in Beispiel 5 angegeben, enthaltend 10»g pRSVL und entweder einen 2:1 oder 3:1 Massenüberschuß an antiCD4-Polylysin90 (s. Beispiel 1) oder gp120-Polylysin (s. Beispiel 3), wie in Fig.2 angegeben, wurden in Abwesenheit oder Gegenwart von 100»M Chloroquin den Zellen hinzugefügt. Nach weiteren 4 h bei 37°C wurden die Proben mit Chloroquin zweimal mit Ham's Medium, enthaltend 10% fötales Kälberserum gewaschen, während den Proben ohne Chloroquin 5 ml desselben Mediums zugefügt wurden. Die Zellen wurden weitere 20 h bei 37°C inkubiert und Aliquots auf Luciferaseaktivität untersucht, wie in Beispiel 6 angegeben. Das Ergebnis dieser Versuche zeigt Fig. 2.

### Beispiel 8

### Import von gp120-Polylysin/pRSVL-Komplexen in CD4⁺ CHO-Zellen

### a) Herstellung von gp120-Polylysin/DNA-Komplexen

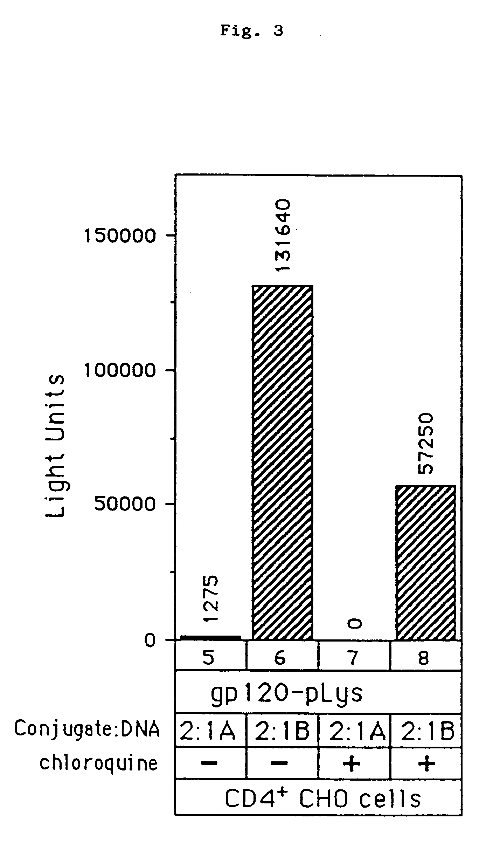

Die Komplexe wurden hergestellt, indem zunächst 6 »g DNA in 330 »l HBS bei Raumtemperatur verdünnt wurden (100 »g/ml oder weniger). Als DNA wurde pRSVL Plasmid-DNA verwendet (vgl. Beispiel 5). Aliquots der in Beispiel 3 erhaltenen gp120-pL190 Konjugate (die Mengen sind in Fig. 3 angegeben) wurden in 170 »l HBS verdünnt. Die jeweilige Konjugatverdünnung wurde rasch der DNA-Verdünnung hinzugefügt, 30 min inkubieren gelassen und anschließend für die Transfektion verwendet.

### b) Transfektion von CD4⁺ Zellen

CD4⁺ CHO-Zellen (Lasky et al., 1987) wurden zu 6x10⁵ Zellen pro T-25-Flasche in Ham's F-12-Medium (Ham, 1965) plus 10 % FCS (fötales Kälberserum) ausgesät.
18 h später wurden die Zellen zweimal mit Ham's F-12-Medium ohne Serum gewaschen und in diesem Medium (5 ml) 5 h lang bei 37°C bebrütet. Darauf hin wurden die Lösungen der gp120-pL/pRSVL Komplexe den Zellen beigegeben. Nach 4 Stunden wurde ein gleiches Volumen DME-Medium (Dulbecco's modified Eagle's Medium) mit einem Gehalt von 10 % fötalem Kälberserum zu jeder Probe gegeben. Nach 24 Stunden wurden die Zellen geerntet, Extrakte hergestellt und Aliquots entsprechend gleichem Proteingehalt (ca. 1/5 des Gesamtmaterials) wie in den vorigen Beispielen auf Luciferaseaktivität untersucht. Die in Fig. 3 angegebenen Werte entsprechen der Luciferaseaktivität von 6 x 10⁵ Zellen. Es wurde festgestellt, daß die Aktivität der gp120-pL Konjugate vom Verhältnis der Komponenten abhängt, wobei größere Aktivität bei den Konjugaten mit niedrigem gp120 : Polylysin-Verhältnis (Fraktion C, Spuren 5 und 6) und eine sehr geringe oder keine Aktivität bei der Fraktion mit einem hohen gp120 : Polylysin-Verhältnis (Fraktion A, Spuren 1 und 2) festgestellt wurde.

### Beispiel 9

### Import von gp120-Polylysin/pRSVL-Komplexen, enthaltend nicht-kovalent gebundenes Polylysin, in CD4⁺ CHO-Zellen

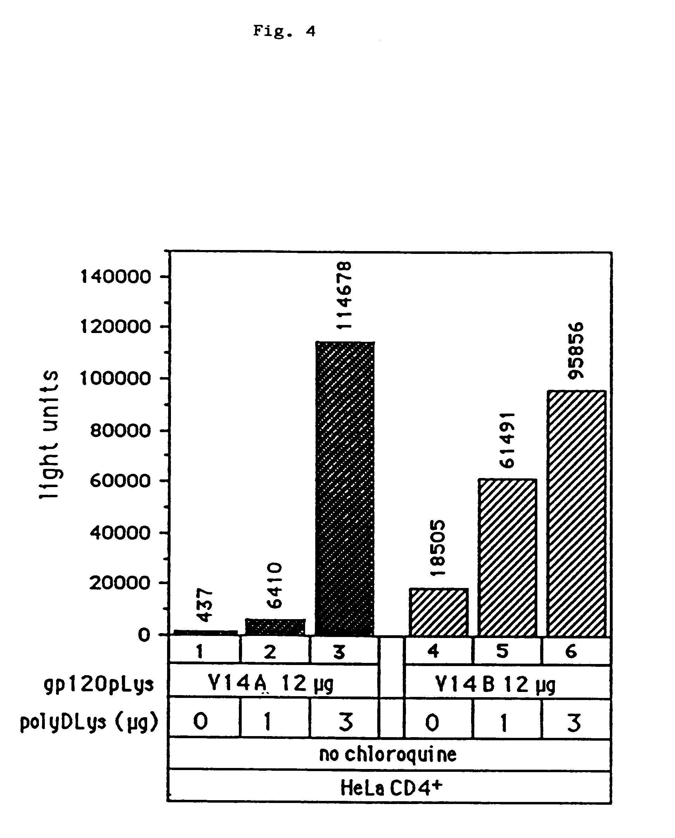

Diejenigen gp120-pL Konjugate, die im Beispiel 8 schlechte Ergebnisse bei der Transfektion gezeigt hatten (Fraktionen A und B) wurden daraufhin untersucht, ob ein Zusatz von freiem Polylysin die DNA-Aufnahme verbessern kann. Es wurden 6 »g DNA und 12 »g Konjugat verwendet, wobei dem Konjugat jeweils vor der Komplexierung mit DNA 1 oder 3 »g Polylysin240 zugesetzt wurden. Übereinstimmend mit den für die Transferrin-Konjugate erhaltenen Ergebnissen wurde eine starke Zunahme der Luciferase-Aktivität (260-fach oder 5,2-fach) beobachtet (Fig. 4).

### Beispiel 10

### Import von gp120-Polylysin/pRSVL-Komplexen in CD4⁺ HeLa-Zellen

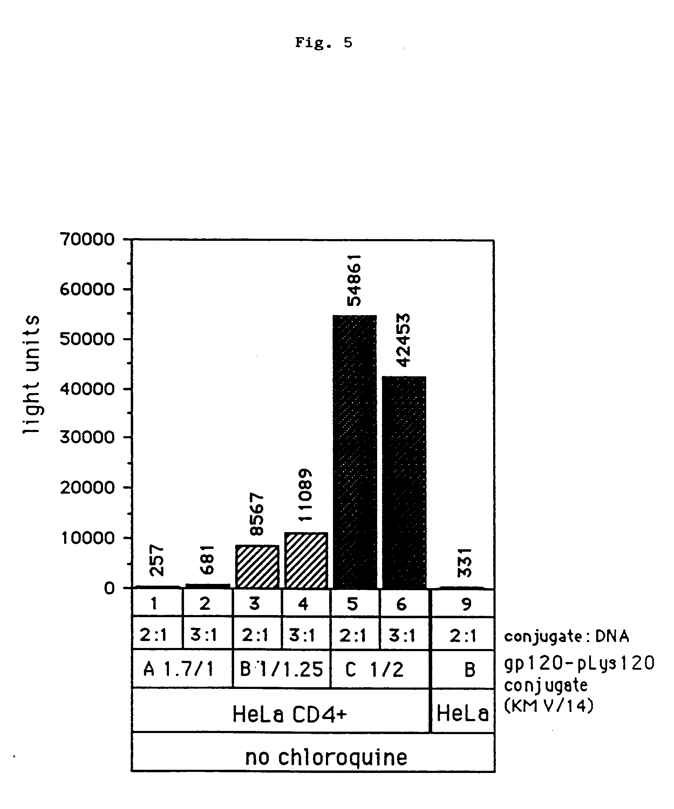

CD4⁺HeLa Zellen (Maddon et al., 1986) oder normale HeLa-Zellen als Kontrolle in DME-Medium plus 10 % FCS wurden zu 6x10⁵ Zellen pro T25-Flasche ausgesät und anschließend gezüchtet, wie in Beispiel 6 für CHO-Zellen angegeben.
Die Zellen wurden mit gp120-Polylysin/pRSVL-Komplexen bei den angegebenen Konjugat/DNA-Verhältnissen (Fig.5) in Berührung gebracht (bei den gp120-Polylysin-Konjugaten A, B und C handelt es sich um drei Fraktionen einer Mono S-Auftrennung des konjugierten Materials aus Beispiel 3b)). Das molare gp120 : Polylysin-Verhältnis jeder Fraktion ist in der Figur angegeben. Nach 4stündigem Kontakt mit den Konjugaten in Abwesenheit von Serum wurde Serum enthaltendes Medium zugesetzt und die Zellen nach 20 h geerntet.
Aus den Zellextrakten wurden Aliquots, standardisiert auf gleichen Proteingehalt, auf Luciferase-Aktivität untersucht. Die in der Figur angegebenen Werte entsprechen der Luciferase-Aktivität von 6x10⁵ Zellen, transfiziert mit 6 »g DNA.

### Beispiel 11

### Transfer und Expression von DNA in H9-Zellen mittels antiCD7-Polylysin190-Konjugaten

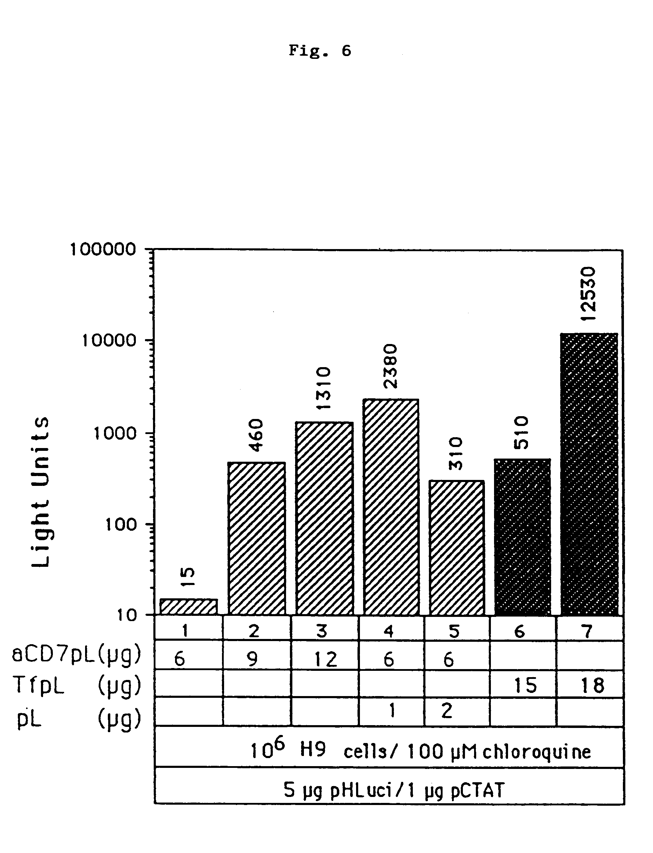

a) Zellen der T-Zellinie H9 (Mann et al., 1989) wurden in RPMI 1640 Medium, ergänzt mit 20 % FCS, 100 Einheiten/ml Penicilin, 100 »g/ml Streptomycin und 1 mM Glutamin gezüchtet. Unmittelbar vor der Transfektion wurden die Zellen durch Zentrifugation gesammelt und zu 100.000 Zellen/ml in frisches Medium aufgenommen (1,000.000 Zellen pro Probe) die zur Transfektion verwendet wurden. Zum Vergleich mit antiCD7-Konjugaten wurden Transferrin-Konjugate verwendet. Transferrin-Polylysin-Konjugate wurden hergestellt, wie in der EP-A 1 388 758 beschrieben; als antiCD7-Konjugate wurden die in Beispiel 4 beschriebenen verwendet. Die Komplexierung mit DNA wurde vorgenommen, wie in Beispiel 5 angegeben. Für die transiente Transfektion in H9-Zellen wurde als DNA das Plasmid pHLuci verwendet, das die HIV-LTR-Sequenz, verbunden mit der für Luciferase codierenden Sequenz, gefolgt von der SV40-Intron/polyA-Stelle, enthält: Das HindIII-Fragment, enthaltend das Protease 2A-Gen, von pHIV/2A (Sun und Baltimore, 1989) würde entfernt und durch ein HindIII/SmaI-Fragment von pRSVL (De Wet et al., 1987), enthaltend die für Luciferase codierende Sequenz, ersetzt. Die beiden Fragmente wurden über die HindIII-Stellen (nach Herstellen glatter Enden mittels Klenow-Fragment) verbunden und dann über die glatte SMA1-Stelle mit der nunmehr glatten HindIII-Stelle verbunden. Ein Klon mit der richtigen Orientierung der Luciferase-Gensequenz wurde ausgewählt. Dieses Plasmid benötigt für starke Transkriptionsaktivität das TAT-Gen-Produkt. Dieses wird durch Co-Transfektion mit dem Plasmid pCMVTAT, welches für das HIV-TAT-Gen unter der Kontrolle des CMV immediate early promoter codiert (Jakobovits et al., 1990) bereitgestellt. Die für die Transfektion verwendeten DNA-Komplexe enthielten eine Mischung von 5 »g pHLuci und 1 »g pCMVTAT. Die DNA/Polykation-Komplexe (500 »l) wurden zu der 10 ml Zellprobe hinzugegeben und 4 h lang in Gegenwart von 100 »M Chloroquin inkubiert. Anschließend wurden die Zellen in frisches Medium gewaschen, 40 h später geerntet und auf Luciferase-Aktivität untersucht, wie in den vorangegangenen Beispielen angegeben. Die Ergebnisse (in Luciferase-Lichteinheiten) sind in Fig. 6 dargestellt: Es zeigte sich, daß die Luciferase-Aktivität mit steigenden Mengen von antiCD7-Polylysin-Konjugat, komplexiert mit 6 »g DNA, zunimmt (Proben 1, 2 und 3). Eine weitere Steigerung der Aktivität wurde beobachtet, wenn 6 »g Konjugat zusammen mit 1 »g freiem Polylysin zur Komplexbildung verwendet wurden (Probe 4), ein weiterer Zusatz von Polylysin beeinträchtigte den Gen-Transport (Probe 5).( Die mit Transferrin-Polylysin-Konjugaten durchgeführten Vergleichsversuche sind mit 6 und 7 bezeichnet.)
b) Eine weitere Versuchsreihe zur Transfektion mit Hilfe der Antikörper-Konjugate wurde unter Verwendung des Plasmids pSSTNEO durchgeführt. Dieses Plasmid, das ein Neomycin-Resistenz-Gen als Marker enthält, wurde unter Verwendung von antiCD4-, antiCD7- und (zum Vergleich) Transferrin-Polylysin190-Konjugaten in H9-Zellen eingeführt (es wurden 6 »g DNA pro 10⁶ Zellen verwendet; die optimalen Transfektionsbedingungen waren in Vorversuchen mit Hilfe transienter Luciferase - Assays ermittelt worden). Das Plasmid pSSTNEO enthält das große Sst-Fragment des pUCu-Locus (Collis et al., 1990), der die HSV TK-neo-Einheit enthält. Ein 63 bp-Fragment, enthaltend eine einzige NdeI-Stelle war in die Asp718-Stelle eingeführt worden. Aliquots der transfizierten Zellen (enthaltend eine definierte Zellzahl) wurden daraufhin in ein halbfestes Methylcellulose-Medium, enthaltend 1000 »g/ml G418, verdünnt. Dazu wurden Aliquots der Zellen 3 Tage nach Transfektion mit DNA, enthaltend den Neomycin-Marker, in ein halbfestes Medium, das zusätzlich zu den normalen Anforderungen 0,5 - 1 mg/ml G418 und 20 mg/ml Methylcellulose enthielt, ausplattiert. (Zur Herstellung des halbfesten Selektionsmediums wurde unter sterilen Bedingungen eine Lösung von 20 g Methylcellulose in 460 ml Wasser hergestellt. Daraufhin wurden 500 ml doppelt konzentriertes, ergänztes Nährmedium, ebenfalls hergestellt unter sterilen Bedingungen, mit der Methylcellulose-Lösung vereinigt, das Volumen auf 1 l gebracht und das Medium über Nacht bei 4°C gerührt. 50 ml Aliquots dieses Mediums wurden, gegebenenfalls nach Lagerung bei -20°C, mit je 10 ml Serum versetzt und das Volumen mit Komplettmedium ohne Serum auf 100 ml gebracht. In diesem Schritt wurde G418 hinzugefügt. Ein 2,5 ml Aliquot des Methylcellulose-Mediums wurde mit einem 50 bis 100 »l Aliquot der Zellsuspension vermischt und ca. 1 ml dieser Mischung in Kulturschalen ausgegossen.) Die Bebrütung erfolgte bei 37°C unter CO₂-Atmosphäre. Ca. 10 bis 14 Tage später wurden die G418-resistenten Zellen gezählt (nur Kolonien mit mehr als 200 Zellen wurden als positiv gewertet). Die Ergebnisse sind in Fig.4 dargestellt (es ist die Zahl von G418 resistenten Kolonien/1000 Zellen 10 Tage nach Einbringen in das Antibiotika-Medium aufgetragen).

### Beispiel 12

### Herstellung von Protein-A-Polylysin190-Konjugaten

Eine Lösung von 4,5 mg Protein-A (Pierce, No. 21182, 107 nmol) in 0,5 ml 100 mM HEPES pH 7,9 wurde mit 30 »l 10 mM ethanolischer Lösung von SPDP (Pharmacia) versetzt. Nach 2 h bei Raumtemperatur wurde über eine Gelsäule Sephadex G 25 filtriert (Eluens 50 mM HEPES-Puffer pH 7,9) wobei 3,95 mg (94 nmol) Protein-A, modifiziert mit 245 nmol Pyridyldithiopropionatresten, erhalten wurden. Poly(L)lysin190, Fluoreszenzmarkierung mittels FITC, wurde analog mit SPDP modifiziert und durch Behandlung mit Dithiothreitol und nachfolgender Gelfiltration in die mit freien Mercaptogruppen modifizierte Form gebracht.
Eine Lösung von 53 nmol Polylysin190, modifiziert mit 150 nmol Mercaptogruppen, in 0,8 ml 30 mM Natriumacetatpuffer wurde unter Sauerstoffausschluß mit oben angeführtem modifiziertem Protein-A gemischt und über Nacht bei Raumtemperatur stehen gelassen. Das Reaktionsgemisch wurde durch Zugabe von 5 M NaCl auf einen Gehalt von ungefähr 0,6 M gebracht. Die Isolierung der Konjugate erfolgte durch Ionenaustauschchromatographie (Mono S, Pharmacia, 50 mM HEPES pH 7,3, Salzgradient 0,6 M bis 3 M NaCl); nach Fraktionierung und Dialyse gegen 25 mM HEPES pH 7,3 wurden zwei Konjugatfraktionen A und B, bestehend aus 1,15 mg (27 nmol) Protein-A, modifiziert mit 5 nmol Polylysin190 (im Falle der Fraktion A) bzw. 2,6 mg (6,2 nmol) Protein-A, modifiziert mit 40 nmol Polylysin190 (Fraktion B), erhalten. Die Komplexe mit DNA wurden analog Beispiel 5 hergestellt.

### Beispiel 13

### Transfektion von CD4⁺ Zellen mit Protein A-Polylysin-Konjugaten

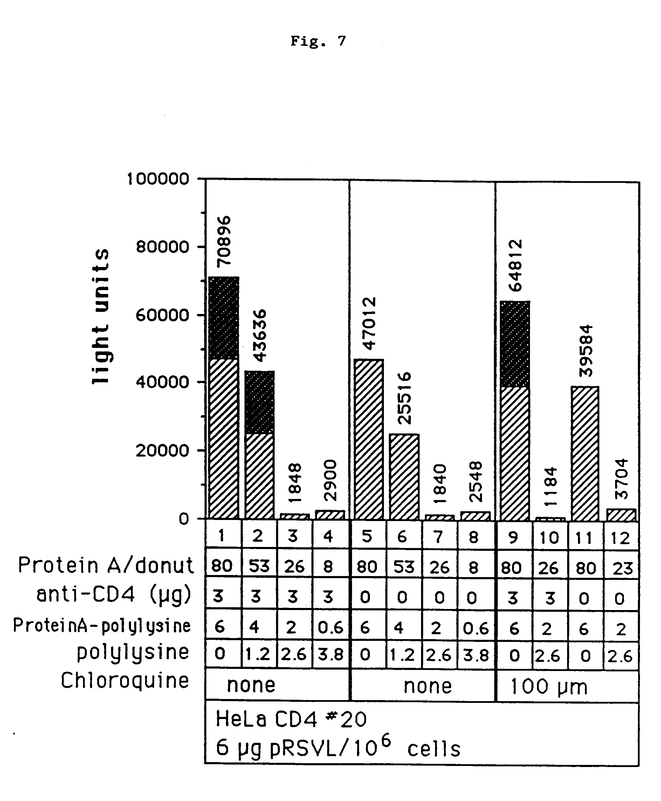

CD4-exprimierende HeLa-Zellen (s. Beispiel 10) wurden zu 6 x 10⁵ Zellen/T25-Flasche ausgesät und anschließend in DME-Medium plus 10 % FCS gezüchtet. Wo in Fig. 7 angegeben, wurden die Zellen mit dem Antikörper (anti-CD4gp55kD, IOT4, Immunotech) (3 »g/Probe) 1 h lang bei Raumtemperatur vorinkubiert. In der Zwischenzeit wurden Protein A-Polylysin190/DNA-Komplexe in 500 »l HBS, enthaltend 6 »g pRSVL und die angegebenen Mengen Protein A-Polylysin190 plus zusätzliches freies Polylysin, wie in Beispiel 5 hergestellt. Nach Ende der 1stündigen Inkubation wurden die Zellen in 4,5 ml frisches Medium gegeben und die 500 »l DNA-Probe bei 37°C zu den Zellen zugegeben. Nach 4 h wurden diejenigen Proben, die 100 »M Chloroquin enthielten (Proben 9-12) in frisches Medium gewaschen, während die Proben 1-8 bis zur Ernte mit der DNA inkubiert wurden. Für den Luciferase-Assay wurden die Zellen 20 h später geerntet. Die Ergebnisse der Versuche sind in Fig. 7 dargestellt. Es zeigte sich, daß die Luciferase-Aktivität von der Gegenwart von Protein A-Polylysin im DNA-Komplex abhängig war (Proben 1-4, 5, 6). Bei den Proben 5-8, 11, 12 wurde DNA-Transport mittels des Protein A-Komplexes ohne Antikörper-Vorbehandlung nachgewiesen; der DNA-Import wurde jedoch um ca. 30 % gesteigert, wenn die Zellen mit dem Antikörper, der das Zelloberflächenprotein CD4 erkennt, vorbehandelt wurden (Proben 1-4, 9, 10). Es wurde weiters festgestellt, daß die Anwesenheit von Chloroquin keine Steigerung der DNA-Expression hervorruft (vgl. die Proben 1-8 mit den Proben 9-12).

### Literatur:

Aruffo A. und Seed B., 1987, EMBO J. 6, No. 11, 3313-3316
Baltimore D., 1988, Nature 335, 395
Chaudhary V.K. et al., 1990, Proc.Natl.Sci.USA 87, 1066
Collis P. et al., 1990, EMBO J. 9, 233-240.
Dalgleish A.G. et al., 1984, Nature 312, 763
De Wet et al., 1987, Mol.Cell.Biol. 7, 725-737
Fauci A., 1988, Science 239, 617
Felsenfeld et al., 1978, Nature 271, 115
Fujiwara et al., 1981, J.Immunol.Meth. 45, 195
Green M. et al., 1989, Cell 58, 215-223
Ham R.G., 1965, Proc.Natl.Acad.Sci.USA 53, 288
Herskowitz I., 1987, Nature 329, 219
Jakobovits A. et al., 1990, EMBO J. 9, 1165-1170
Johnston S.A., 1990, Nature 346, 776-777
Jung et al., 1981, Biochem.Res.Commun.101, 599
Kasid A. et al., 1990, Proc.Natl.Acad.Sci.USA 87, 473-477
Klatzmann D. et al., 1984, Nature 312, 767
Lasky et al., 1986, Science 233, 209-212
Lasky et al., 1987, Cell 50, 975-985
Luthmann und Magnussen, 1983, Nucl.Acids Res.11 1295-1308
Maddon P.J. et al., 1986, Cell 47, 333-348
Malim M. et al., 1989, Cell 58, 205-214
Maniatis, 1982, Molecular Cloning, Cold Spring Harbor
Mann D.L. et al., 1989, AIDS Research and Human Retroviruses, 5, 253
McDougal J.S. et al., 1986, Science 231, 382
Nabel E.G. et al., 1990, Science 249, 1285-1288
Nygren A. et al., 1988, Proc.Natl.Acad.Sci.USA 85, 6543-6546
Orlandi R. et al., 1989, Proc.Natl.Acad.Sci.USA 86, 3833-3837
Pauza C.D. und Price T.M., 1988, J.Cell Biol. 107, 959-968
Pelchen-Matthews et al., 1989, EMBO J.8, 3641-3649
Sastry L. et al., 1989, Proc.Natl.Acad.Sci.USA 86, 5728-5732
Smith D.H. et al., 1987, Science 238, 1704-1707
Stein B.S. et al., 1987, Cell 49, 659
Surolia A. et al., 1982, TIBS 7, 74-75
Trono D. et al., 1989, Cell 59, 113-120
Warrant R. et al., 1978, Nature 271, 130-135
Wilson J.M. et al., 1990, Proc.Natl.Acad.Sci.USA 87, 439-443
Wu G.Y. und Wu C.H., 1987, J.Biol.Chem. 263, 14621-14624
Yang N.S. et al., 1990, Proc.Natl.Acad.Sci.USA 87, 9568-9572
Zamecnik et al., 1986, Proc.Natl.Acad.Sci.USA 83, 4143
Zon G., 1988, Pharmaceut. Research 5, No.9, 539-549

## Patentansprüche

1. Protein-Polykation-Konjugate, die befähigt sind, mit Nukleinsäuren oder Nukleinsäureanalogen lösliche Komplexe zu bilden, die in menschliche oder tierische Zellen aufgenommen werden, dadurch gekennzeichnet, daß der Proteinanteil der Konjugate ein Protein mit der Fähigkeit ist, an ein von Zellen der T-Zell-Abstammungslinie exprimiertes Zelloberflächenprotein zu binden, so daß die gebildeten Komplexe in Zellen, die das T-Zelloberflächenprotein exprimieren, aufgenommen werden.

2. Konjugate nach Anspruch 1, dadurch gekennzeichnet, daß ihr Proteinanteil ein, vorzugsweise monoklonaler, Antikörper, bzw. ein Fragment davon, ist, gerichtet gegen ein T-Zelloberflächenprotein.

3. Konjugate nach Anspruch 1 oder 2, dadurch gekennzeichnet, daß sie ein Protein mit der Fähigkeit enthalten, an CD4 zu binden.

4. Konjugate nach Anspruch 3, dadurch gekennzeichnet, daß sie einen monoklonalen anti-CD4-Antikörper bzw. das Fragment davon enthalten, das ein gp120 bindendes Epitop aufweist.

5. Konjugate nach Anspruch 3, dadurch gekennzeichnet, daß sie als Protein HIV-1 gp120 bzw. ein homologes Protein verwandter Retroviren oder ein Fragment davon enthalten, das an CD4 bindet.

6. Konjugate nach Anspruch 2, dadurch gekennzeichnet, daß sie als Protein einen anti-CD3-Antikörper enthalten.

7. Konjugate nach Anspruch 1 oder 2, dadurch gekennzeichnet, daß sie ein Protein enthalten, das an einen auf T-Zellen exprimierten Tumormarker bindet.

8. Konjugate nach Anspruch 7, dadurch gekennzeichnet, daß sie ein Protein enthalten, das an CD7 bindet.

9. Konjugate nach einem der Ansprüche 2, 4, 7 oder 8, dadurch gekennzeichnet, daß sie einen Antikörper in direkt an das Polykation gekoppelter Form enthalten.

10. Konjugate nach einem der Ansprüche 2, 4, 7 oder 8, dadurch gekennzeichnet, daß sie einen Antikörper in über an Polykation gekoppeltes Protein A gebundener Form enthalten.

11. Protein A-Polykation-Konjugate zur Herstellung von Antikörper-Konjugaten nach Anspruch 10.

12. Konjugate nach Anspruch 1, dadurch gekennzeichnet, daß das Polykation ein, gegebenenfalls modifiziertes, Protamin ist.

13. Konjugate nach Anspruch 1, dadurch gekennzeichnet, daß das Polykation ein, gegebenenfalls modifiziertes, Histon ist.

14. Konjugate nach Anspruch 1, dadurch gekennzeichnet, daß das Polykation ein synthetisches homologes oder heterologes Polypeptid ist.

15. Konjugate nach Anspruch 14, dadurch gekennzeichnet, daß das Polypeptid Polylysin ist.

16. Konjugate nach einem der Ansprüche 12 bis 15, dadurch gekennzeichnet, daß das Polykation ca. 20 bis 500 positive Ladungen aufweist.

17. Konjugate nach einem der Ansprüche 12 bis 16, dadurch gekennzeichnet, daß das molare Verhältnis T-Zell-bindendes Protein:Polykation etwa 10:1 bis 1:10 beträgt.

18. Neue Protein-Polykation/Nukleinsäure-Komplexe, die in menschliche oder tierische Zellen aufgenommen werden, dadurch gekennzeichnet, daß der Proteinanteil der Konjugate ein Protein mit der Fähigkeit ist, an ein von Zellen der T-Zell-Abstammungslinie exprimiertes Zelloberflächenprotein zu binden, so daß die gebildeten Komplexe in Zellen, die das T-Zelloberflächenprotein exprimieren, aufgenommen werden.

19. Komplexe nach Anspruch 18, dadurch gekennzeichnet, daß sie als Konjugat-Anteil eines der in den Ansprüchen 1 bis 10 oder 12 bis 17 definierten Konjugate enthalten.

20. Komplexe nach einem der Ansprüche 18 oder 19, dadurch gekennzeichnet, daß sie zusätzlich ein nicht-kovalent gebundenes Polykation, das gegebenenfalls identisch ist mit dem Polykation des Konjugats, derart enthalten, daß die durch das Konjugat erzielte Internalisierung und/oder Expression der Nukleinsäure gesteigert wird.

21. Komplexe nach einem der Ansprüche 18 bis 20, dadurch gekennzeichnet, daß sie eine virusinhibierende Nukleinsäure enthalten.

22. Komplexe nach Anspruch 21, dadurch gekennzeichnet, daß sie eine Nukleinsäure enthalten, die Replikation und Expression des HIV-1 Virus oder verwandter Retroviren inhibiert.

23. Komplexe nach Anspruch 22, dadurch gekennzeichnet, daß die inhibierende Nukleinsäure Komplementarität zu Sequenzen des HIV-1-Genoms aufweist.

24. Komplexe nach Anspruch 23, dadurch gekennzeichnet, daß die Nukleinsäure Komplementarität zu Sequenzen des tat-Gens aufweist.

25. Komplexe nach Anspruch 23, dadurch gekennzeichnet, daß die Nukleinsäure Komplementarität zu Sequenzen des rev-Gens aufweist.

26. Komplexe nach Anspruch 23, dadurch gekennzeichnet, daß die Nukleinsäure Komplementarität zu Sequenzen des nef-Gens aufweist.

27. Komplexe nach Anspruch 23, dadurch gekennzeichnet, daß die Nukleinsäure Komplementarität zu LTR-Sequenzen aufweist.

28. Komplexe nach Anspruch 23, dadurch gekennzeichnet, daß die Nukleinsäure Komplementarität zur tar-Sequenz aufweist.

29. Komplexe nach einem der Ansprüche 21 bis 28, dadurch gekennzeichnet, daß sie eine inhibierende Nukleinsäure in Form eines Ribozyms, gegebenenfalls zusammen mit einer Carrier-RNA, bzw. des dafür codierenden Gens enthalten.

30. Komplexe nach Anspruch 29, dadurch gekennzeichnet, daß sie eine Nukleinsäure in Form einer genetischen Einheit, bestehend aus einem tRNA-Gen als Carrier-Gen und einem innerhalb dieses Gens angeordneten Ribozym-Gen, enthalten.

31. Komplexe nach einem der Ansprüche 21 bis 28, dadurch gekennzeichnet, daß sie eine inhibierende Nukleinsäure in Form eines, gegebenenfalls modifizierten, Antisense-Oligonukleotids, gegebenenfalls zusammen mit einer Carrier-Nukleinsäure, bzw. im Fall eines RNA-Oligonukleotids des dafür codierenden Gens enthalten.

32. Komplexe nach Anspruch 21, dadurch gekennzeichnet, daß sie eine Nukleinsäure, codierend für ein Virusprotein, das eine trans-dominante Mutation aufweist, enthalten.

33. Komplexe nach einem der Ansprüche 18 bis 20, dadurch gekennzeichnet, daß sie eine Oncogen-inhibierende Nukleinsäure enthalten.

34. Komplexe nach Anspruch 33, dadurch gekennzeichnet, daß sie eine Oncogen-inhibierende Nukleinsäure in Form eines Ribozyms, gegebenenfalls zusammen mit einer Carrier-RNA, bzw. des dafür codierenden Gens enthalten.

35. Komplexe nach Anspruch 34, dadurch gekennzeichnet, daß sie eine Oncogen-inhibierende Nukleinsäure in Form eines Ribozyms, gegebenenfalls zusammen mit einer Carrier-RNA, bzw. des dafür codierenden Gens enthalten.

36. Komplexe nach einem der Ansprüche 18 bis 20, dadurch gekennzeichnet, daß sie als Nukleinsäure ein therapeutisch oder gentherapeutisch wirksames Gen bzw. einen Genabschnitt enthalten.

37. Verfahren zum Einführen von Nukleinsäure(n) in Zellen, die ein T-Zelloberflächenprotein exprimieren, wobei man aus einem der in den Ansprüchen 1 bis 10 oder 12 bis 17 definierten Protein-Polykation-Konjugate und Nukleinsäure(n), gegebenenfalls in Gegenwart von nicht-kovalent gebundenem Polykation, einen der in den Ansprüchen 18 bis 36 definierten, vorzugsweise unter physiologischen Bedingungen löslichen, Komplexe bildet und *in vitro* oder *ex vivo* Zellen, die das T-Zelloberflächenprotein exprimieren, insbesondere T-Zellen, gegebenenfalls unter Bedingungen, unter denen der Abbau von Nukleinsäure in der Zelle inhibiert wird, mit diesem Komplex in Berührung bringt.

38. Verfahren nach Anspruch 37, wobei man aus einem Protein A-Polykation-Konjugat, bestehend aus Protein A und einem der in den Ansprüchen 12 bis 16 definierten Polykationen und einer der in den Ansprüchen 21 bis 36 definierten Nukleinsäuren einen Komplex bildet und in Gegenwart eines Antikörpers, gerichtet gegen ein T-Zelloberflächenprotein, mit Zellen, die dieses Oberflächenprotein exprimieren, *in vitro* oder *ex vivo* in Berührung bringt, wobei der Antikörper an den Konjugat-Anteil des Komplexes gebunden wird.

39. Pharmazeutische Zubereitung, enthaltend als wirksame Komponente eine oder mehrere therapeutisch oder gentherapeutisch wirksame Nukleinsäure(n) als Bestandteil eines in einem der Ansprüche 18 bis 36 definierten Komplexes.

## Claims

1. Protein-polycation conjugates which are capable of forming, with nucleic acids or nucleic acid analogues, soluble complexes which are absorbed into human or animal cells, characterised in that the protein component of the conjugates is a protein capable of binding to a cell surface protein expressed by cells of the T-cell lineage, so that the complexes formed are taken up into cells which express the T-cell surface protein.

2. Conjugates according to claim 1, characterised in that their protein component is a preferably monoclonal antibody or a fragment thereof, directed against the T-cell surface protein.

3. Conjugates according to claim 1 or 2, characterised in that they contain a protein capable of binding to CD4.

4. Conjugates according to claim 3, characterised in that they contain a monoclonal anti-CD4 antibody or the fragment thereof which contains a gp120 binding epitope.

5. Conjugates according to claim 3, characterised in that they contain as protein HIV-1 gp120 or a homologous protein of related retroviruses or a fragment thereof which binds to CD4.

6. Conjugates according to claim 2, characterised in that they contain an anti-CD3-antibody as protein.

7. Conjugates according to claim 1 or 2, characterised in that they contain a protein which binds to a tumour marker expressed on T-cells.

8. Conjugates according to claim 6, characterised in that they contain a protein which binds to CD7.

9. Conjugates according to one of claims 2, 4, 7 or 8, characterised in that they contain an antibody in a form which is directly coupled to the polycation.

10. Conjugates according to one of claims 2, 4, 7 or 8, characterised in that they contain an antibody in a form bound by means of a protein A coupled to polycation.

11. Protein A-polycation conjugates for preparing antibody conjugates according to claim 10.

12. Conjugates according to claim 1, characterised in that the polycation is an optionally modified protamine.

13. Conjugates according to claim 1, characterised in that the polycation is an optionally modified histone.

14. Conjugates according to claim 1, characterised in that the polycation is a synthetic homologous or heterologous polypeptide.

15. Conjugates according to claim 14, characterised in that the polypeptide is polylysine.

16. Conjugates according to one of claims 12 to 15, characterised in that the polycation has about 20 to 500 positive charges.

17. Conjugates according to one of claims 12 to 16, characterised in that the molar ratio of T-cell binding protein to polycation is about 10:1 to 1:10.

18. New protein-polycation/nucleic acid complexes which are absorbed into human or animal cells, characterised in that the protein component of the conjugates is a protein capable of binding to a cell surface protein expressed by cells of the T-cell lineage, so that the complexes formed are taken up in cells which express the T-cell surface protein.

19. Complexes according to claim 18, characterised in that they contain as the conjugate component one of the conjugates defined in claims 1 to 10 or 12 to 17.

20. Complexes according to one of claims 18 or 19, characterised in that they additionally contain a non-covalently bound polycation, which may optionally be identical to the polycation of the conjugate, so that the internalisation and/or expression of the nucleic acid achieved by the conjugate is increased.

21. Complexes according to one of claims 18 to 20, characterised in that they contain a virus inhibiting nucleic acid.

22. Complexes according to claim 21, characterised in that they contain a nucleic acid which inhibits replication and expression of the HIV-1 virus or related retroviruses.

23. Complexes according to claim 22, characterised in that the inhibiting nucleic acid is complementary to sequences of the HIV-1 genome.

24. Complexes according to claim 23, characterised in that the nucleic acid is complementary to sequences of the tat gene.

25. Complexes according to claim 23, characterised in that the nucleic acid is complementary to sequences of the rev gene.

26. Complexes according to claim 23, characterised in that the nucleic acid is complementary to sequences of the nef gene.

27. Complexes according to claim 23, characterised in that the nucleic acid is complementary to LTR-sequences.

28. Complexes according to claim 23, characterised in that the nucleic acid is complementary to the tar sequence.

29. Complexes according to one of claims 21 to 28, characterised in that they contain an inhibiting nucleic acid in the form of a ribozyme, optionally together with a carrier RNA, or the gene coding therefor.

30. Complexes according to claim 29, characterised in that they contain a nucleic acid in the form of a genetic unit consisting of a tRNA-gene as carrier gene and a ribozyme gene arranged within this gene.

31. Complexes according to one of claims 21 to 28, characterised in that they contain an inhibiting nucleic acid in the form of an optionally modified antisense oligonucleotide, optionally together with a carrier nucleic acid, or in the case of an RNA-oligonucleotide, the gene coding therefor.

32. Complexes according to claim 21, characterised in that they contain a nucleic acid coding for a virus protein which contains a trans-dominant mutation.

33. Complexes according to one of claims 18 to 20, characterised in that they contain an oncogene-inhibiting nucleic acid.

34. Complexes according to claim 33, characterised in that they contain an oncogene-inhibiting nucleic acid in the form of a ribozyme, optionally together with a carrier RNA or the gene coding therefor.

35. Complexes according to claim 34, characterised in that they contain an oncogene-inhibiting nucleic acid in the form of a ribozyme, optionally together with a carrier RNA, or the gene coding therefor.

36. Complexes according to one of claims of 18 to 20, characterised in that they contain as nucleic acid a therapeutically or gene therapeutically active gene or gene section.

37. Process for introducing nucleic acid or acids into cells which express a T-cell surface protein, by forming one of the complexes defined in claims 18 to 36, which is preferably soluble under physiological conditions, from one of the protein-polycation conjugates defined in claims 1 to 10 or 12 to 17 and nucleic acid or acids, optionally in the presence of non-covalently bound polycation, and, in vitro or ex vivo, bringing cells which express the T-cell surface protein, especially T-cells, into contact with this complex, optionally under conditions under which the breakdown of nucleic acid in the cell is inhibited.

38. Process according to claim 37, in which a complex is formed from a protein A-polycation conjugate, consisting of protein A and one of the polycations defined in claims 12 to 16 and one of the nucleic acids defined in claims 21 to 36, and the complex is brought into contact, in the presence of an antibody directed against a T-cell surface protein, with cells which express this surface protein, in vitro or ex vivo, the antibody being bound to the conjugate component of the complex.

39. Pharmaceutical preparation containing as active component one or more therapeutically or gene therapeutically active nucleic acids in the form of one of the complexes defined in claims 18 to 36.

## Revendications

1. Conjugués protéine-polycation qui sont capables de former avec des acides nucléiques ou avec des analogues d'acides nucléiques des complexes solubles qui sont absorbés dans des cellules humaines ou animales, caractérisés en ce que la partie protéique des conjugués est une protéine ayant la capacité de se lier à une protéine de surface cellulaire exprimée par des cellules de la lignée de descendance des cellules T, de sorte que les complexes formés sont absorbés dans des cellules qui expriment la protéine de surface de cellule T.

2. Conjugués selon la revendication 1, caractérisés en ce que leur partie protéique est un anticorps, de préférence monoclonal, ou un fragment de celui-ci, dirigé contre une protéine de surface de cellule T.

3. Conjugués selon la revendication 1 ou 2, caractérisés en ce qu'ils contiennent une protéine ayant la capacité de se lier à CD4.

4. Conjugués selon la revendication 3, caractérisés en ce qu'ils contiennent un anticorps monoclonal anti-CD4, ou son fragment qui présente un épitope qui se lie à gp120.

5. Conjugués selon la revendication 3, caractérisés en ce qu'ils contiennent comme protéine gp120 de VIH-1 ou une protéine homologue de rétrovirus apparentés ou un fragment de celle-ci qui se lie à CD4.

6. Conjugués selon la revendication 2, caractérisés en ce qu'ils contiennent comme protéine un anticorps anti-CD3.

7. Conjugués selon la revendication 1 ou 2, caractérisés en ce qu'ils contiennent une protéine qui se lie à un marqueur tumoral exprimé sur des cellules T.

8. Conjugués selon la revendication 7, caractérisés en ce qu'ils contiennent une protéine qui se lie à CD7.

9. Conjugués selon l'une des revendications 2, 4, 7 ou 8, caractérisés en ce qu'ils contiennent un anticorps sous forme couplée directement au polycation.

10. Conjugués selon l'une des revendications 2, 4, 7 ou 8, caractérisés en ce qu'ils contiennent un anticorps sous forme liée par l'intermédiaire de la protéine A couplée à un polycation.

11. Conjugués protéine A-polycation pour la préparation de conjugués d'anticorps selon la revendication 10.

12. Conjugués selon la revendication 1, caractérisés en ce que le polycation est une protamine éventuellement modifiée.

13. Conjugués selon la revendication 1, caractérisés en ce que le polycation est une histone éventuellement modifiée.

14. Conjugués selon la revendication 1, caractérisés en ce que le polycation est un polypeptide homologue ou hétérologue synthétique.

15. Conjugués selon la revendication 14, caractérisés en ce que le polypeptide est la polylysine.

16. Conjugués selon l'une des revendications 12 à 15, caractérisés en ce que le polycation présente environ 20 à 500 charges positives.

17. Conjugués selon l'une des revendications 12 à 16, caractérisés en ce que le rapport molaire protéine se liant aux cellules T:polycation est d'environ 10:1 à 1:10.

18. Nouveaux complexes protéine-polycation/acide nucléique qui sont absorbés dans des cellules humaines ou animales, caractérisés en ce que la partie protéique des conjugués est une protéine ayant la capacité de se lier à une protéine de surface cellulaire exprimée par des cellules de la lignée de descendance des cellules T de sorte que les complexes formés sont absorbés dans des cellules qui expriment la protéine de surface de cellule T.

19. Complexes selon la revendication 18, caractérisés en ce qu'ils contiennent comme partie conjugué l'un des conjugués définis dans les revendications 1 à 10 ou 12 à 17.

20. Complexes selon l'une des revendications 18 ou 19, caractérisés en ce qu'ils contiennent en outre un polycation lié de manière non covalente qui est éventuellement identique au polycation du conjugué, de telle manière que l'internalisation et/ou l'expression de l'acide nucléique obtenue avec le conjugué est augmentée.

21. Complexes selon l'une des revendications 18 à 20, caractérisés en ce qu'ils contiennent un acide nucléique inhibiteur de virus.

22. Complexes selon la revendication 21, caractérisés en ce qu'ils contiennent un acide nucléique qui inhibe la réplication et l'expression du virus VIH-1 ou de rétrovirus apparentés.

23. Complexes selon la revendication 22, caractérisés en ce que l'acide nucléique inhibiteur présente une complémentarité avec des séquences du génome de VIH-1.

24. Complexes selon la revendication 23, caractérisés en ce que l'acide nucléique présente une complémentarité avec des séquences du gène tat.

25. Complexes selon la revendication 23, caractérisés en ce que l'acide nucléique présente une complémentarité avec des séquences du gène rev.

26. Complexes selon la revendication 23, caractérisés en ce que l'acide nucléique présente une complémentarité avec des séquences du gène nef.

27. Complexes selon la revendication 23, caractérisés en ce que l'acide nucléique présente une complémentarité avec des séquences de LTR.

28. Complexes selon la revendication 23, caractérisés en ce que l'acide nucléique présente une complémentarité avec la séquence tar.

29. Complexes selon l'une des revendications 21 à 28, caractérisés en ce qu'ils contiennent un acide nucléique inhibiteur sous forme d'un ribozyme, éventuellement conjointement avec un ARN porteur, ou du gène codant celui-ci.

30. Complexes selon la revendication 29, caractérisés en ce qu'ils contiennent un acide nucléique sous forme d'une unité génétique consistant en un gène d'ARNt en tant que gène porteur et en un gène de ribozyme disposé à l'intérieur de ce gène.

31. Complexes selon l'une des revendications 21 à 28, caractérisés en ce qu'ils contiennent un acide nucléique inhibiteur sous forme d'un oligonucléotide antisens éventuellement modifié, éventuellement conjointement avec un acide nucléique porteur, ou, dans le cas d'un oligonucléotide d'ARN, du gène codant celui-ci.

32. Complexes selon la revendication 21, caractérisés en ce qu'ils contiennent un acide nucléique codant une protéine virale qui présente une mutation trans-dominante.

33. Complexes selon l'une des revendications 18 à 20, caractérisés en ce qu'ils contiennent un acide nucléique inhibiteur d'oncogènes.

34. Complexes selon la revendication 33, caractérisés en ce qu'ils contiennent un acide nucléique inhibiteur d'oncogènes sous forme d'un ribozyme, éventuellement conjointement avec un ARN porteur, ou du gène codant celui-ci.

35. Complexes selon la revendication 34, caractérisés en ce qu'ils contiennent un acide nucléique inhibiteur d'oncogènes sous forme d'un ribozyme, éventuellement conjointement avec un ARN porteur, ou du gène codant celui-ci.

36. Complexes selon l'une des revendications 18 à 20, caractérisés en ce qu'ils contiennent comme acide nucléique un gène ou un segment de gène efficace du point de vue thérapeutique ou de la thérapie génique.

37. Procédé pour introduire un ou des acides nucléiques dans des cellules qui expriment une protéine de surface de cellule T, dans lequel on forme l'un des complexes définis dans les revendications 18 à 36, de préférence soluble dans les conditions physiologiques, à partir de l'un des conjugués protéine-polycation définis dans les revendications 1 à 10 ou 12 à 17 et d'un acide nucléique ou d'acides nucléiques, éventuellement en présence d'un polycation lié de manière non covalente, et on met en contact in vitro ou ex vivo avec ce complexe des cellules qui expriment la protéine de surface de cellule T, en particulier des cellules T, éventuellement dans des conditions dans lesquelles la dégradation d'un acide nucléique dans la cellule est inhibée.

38. Procédé selon la revendication 37, dans lequel on forme un complexe à partir d'un conjugué protéine A-polycation consistant en protéine A et en l'un des polycations définis dans les revendications 12 à 16 et de l'un des acides nucléiques définis dans les revendications 21 à 36 et, en présence d'un anticorps dirigé contre une protéine de surface de cellule T, on le met en contact in vitro ou ex vivo avec des cellules qui expriment cette protéine de surface, l'anticorps se liant à la partie conjugué du complexe.

39. Préparation pharmaceutique contenant comme composant actif un ou plusieurs acides nucléiques actifs du point de vue thérapeutique ou de la thérapie génique en tant que constituant d'un complexe défini dans l'une des revendications 18 à 36.
